# EUROPEAN PATENT APPLICATION

(11) **EP 4 059 523 A1**
(43) Date of publication of application: **21.09.2022**
(21) Application number: 20887181.4
(22) Date of filing: 12.11.2020
(51) Int. Cl.: A61K 48/00, A61P 9/04, C12N 15/12, C12N 15/63, A61K 35/76

(54) **ENHANCER POLYNUCLEOTIDE RESPONDING TO HEART FAILURE AND EXPRESSION VECTOR INCLUDING SAID ENHANCER POLYNUCLEOTIDE**

(30) Priority: 15.11.2019 JP 2019206823
(71) Applicant: OSAKA UNIVERSITY, Suita-shi Osaka 565-0871 (JP)
(72) Inventor: TAKASHIMA, Seiji, Suita-shi, Osaka 565-0871 (JP); MATSUOKA, Ken, Suita-shi, Osaka 565-0871 (JP); ASANO, Yoshihiro, Suita-shi, Osaka 565-0871 (JP); TSUKAMOTO, Osamu, Suita-shi, Osaka 565-0871 (JP); KIOKA, Hidetaka, Suita-shi, Osaka 565-0871 (JP)
(74) Representative: Marks & Clerk LLP
(86) International application number: PCT/JP2020/042283
(87) International publication number: WO 2021/095809

(57) **Abstract**

The object of the present invention is to provide a polynucleotide which can be inserted into an expression vector and can enhance the transcriptional activity of a predetermined promoter under heart failure.

The object is achieved by an enhancer polynucleotide comprising a polynucleotide consisting of a sequence having 80% or more identity with a sequence represented by SEQ ID NO: 1, or a polynucleotide consisting of a sequence having 80% or more identity with a sequence represented by SEQ ID NO: 2, wherein the enhancer polynucleotide responds to heart failure (where the enhancer polynucleotide excludes a polynucleotide having the same sequence as a sequence represented by SEQ ID NO: 13 and a polynucleotide having the same sequence as a sequence represented by SEQ ID NO: 12).

## Description

### Technical Field

The present description discloses an enhancer polynucleotide that responds to heart failure, an expression vector including the enhancer, a composition for gene therapy, and a composition for preventing and/or treating heart failure.

### Background Art

Heart failure generally refers to a pathological condition in which cardiac function is impaired due to various causes including cardiomyopathy, hypertension, and ischemic heart disease such as myocardial infarction and angina pectoris. Currently, as biomarkers for diagnosing such heart failure, atrial natriuretic peptide (ANP), brain natriuretic peptide (BNP) and proBNP which is a precursor protein of BNP are known (Non Patent Literature 1). By measuring these markers of a patient, it is possible to know whether or not the patient has heart failure or how severe it is.

In Patent Literature 1 and Non Patent Literature 2, a non-human transgenic animals to which an expression regulatory region of a gene encoding ANP and/or BNP induced in heart failure and a base sequence containing a reporter gene are transferred are disclosed. In Patent Literature 1 and Non Patent Literature 2, the expression regulatory region of the gene encoding ANP and/or BNP induced in heart failure is referred to as a CR9 region.

### Citation List

### Patent Literature

Patent Literature1: Japanese Unexamined Patent Application Publication No. 2015-084764 Non Patent Literature

Non Patent Literature 1: L.S.Nielsen,etal.,;EurJHeartFail, 6 (2004), pp. 63-70
Non Patent Literature 2: Matsuoka K, et al. :FASEB J., 28 (4) (2014), pp. 1870-9., doi: 10.1096/fj.13-245522. Epub 2014 Jan 3.

### Summary of Invention

### Technical Problem

In recent years, gene therapies have been clinically applied one after another. In gene therapy, it is the most important task to express a gene necessary for therapy in a target cell or express it in vivo specific to disease. Further, although viral vectors and the like that infect in the cell-specific manner have been developed for gene therapy, there are cases where the length of a polynucleotide that can be inserted into the viral vector is limited. Therefore, when a target gene inserted into the viral vector is large, it is necessary to make a transcriptional regulatory region expressing the target gene and other regions as short as possible.

An object of the present invention is to provide a polynucleotide that can be inserted into an expression vector and can enhance transcriptional activity of a predetermined promoter under heart failure.

### Solution to Problem

As a result of diligent research, the present inventor has found an enhancer sequence that responds to heart failure superior to the CR9 region reported in Non Patent Literature 2.

The present invention has been achieved based on these above findings and includes following aspects.

### Item 1

An enhancer polynucleotide comprising
a polynucleotide consisting of a sequence having 80% or more identity with a sequence represented by SEQ ID NO: 1, or
a polynucleotide consisting of a sequence having 80% or more identity with a sequence represented by SEQ ID NO: 2
wherein the enhancer polynucleotide responds to heart failure (where the enhancer polynucleotide excludes a polynucleotide having the same sequence as a sequence represented by SEQ ID NO: 13 and a polynucleotide having the same sequence as a sequence represented by SEQ ID NO: 12).

### Item 2

The enhancer polynucleotide according to item 1, wherein
the enhancer polynucleotide comprising the polynucleotide consisting of the sequence having 80% or more identity with the sequence represented by SEQ ID NO: 1 consists of a sequence having 70% or more identity with a sequence represented by SEQ ID NO: 4, or
the enhancer polynucleotide comprising a polynucleotide consisting of the sequence having 80% or more identity with the sequence represented by SEQ ID NO: 2 consists of a sequence having 70% or more identity with a sequence represented by SEQ ID NO: 7.

### Item 3

The enhancer polynucleotide according to item 1 or 2, wherein
(i) the sequence having 80% or more identity with the sequence represented by SEQ ID NO: 1 or
(ii) the sequence having 80% or more identity with the sequence represented by SEQ ID NO: 2
is a sequence represented by SEQ ID NO: 3.

### Item 4

The enhancer polynucleotide according to any one of items 1 to 3, wherein the enhancer polynucleotide further comprises
a polynucleotide consisting of a sequence having 90% or more identity with a sequence represented by SEQ ID NO: 5 or
a polynucleotide consisting of a sequence having 90% or more identity with a sequence represented by SEQ ID NO: 8.

### Item 5

The enhancer polynucleotide according to any one of items 1 to 4, wherein
the enhancer polynucleotide is
A: obtained by connecting (i) the polynucleotide comprising the sequence having 80% or more identity with the sequence represented by SEQ ID NO: 1, and (ii) the polynucleotide consisting of a sequence having 90% or more of the sequence represented by SEQ ID NO: 5 directly or via an adapter, or
B: obtained by connecting (iii) the polynucleotide comprising the sequence having 80% or more identity with the sequence represented by SEQ ID NO: 2 and (iv) the polynucleotide consisting of the sequence having 90% or more identity with the sequence represented by SEQ ID NO: 8 directly or via an adapter, and wherein
the length of the adapter is 10 nucleotides or less when expressed in terms of the number of nucleotides in a single-stranded polynucleotide.

### Item 6

The enhancer polynucleotide according to item 1, wherein the total length of the enhancer polynucleotide is 200 nucleotides or less when expressed in terms of the number of nucleotides in the single-stranded polynucleotide.

### Item 7

An enhancer polynucleotide comprising a polynucleotide consisting of the sequence represented by SEQ ID NO: 10 or SEQ ID NO: 11, wherein the enhancer polynucleotide responds to heart failure.

### Item 8

An enhancer polynucleotide comprising a reverse complementary sequence of the enhancer polynucleotide according to any one of items 1 to 7, wherein the enhancer polynucleotide responds to heart failure.

### Item 9

An expression vector comprising a promoter polynucleotide and the enhancer polynucleotide according to any one of items 1 to 8, wherein the polynucleotide containing a promoter sequence and the enhancer polynucleotide are connected so that the enhancer polynucleotide enhances the transcriptional activity of the promoter polynucleotide.

### Item 10

A composition for gene therapy comprising a polynucleotide in which the polynucleotide to be expressed is integrated into the expression vector according to item 9 so as to be expressible.

### Item 11

The composition according to item 10 for preventing and/or treating heart failure.

### Item 12

A composition for preventing and/or treating heart failure, comprising
a polynucleotide comprising an sgRNA sequence that binds to a polynucleotide consisting of the sequence represented by SEQ ID NO: 1, the sequence represented by SEQ ID NO: 2, the sequence represented by SEQ ID NO: 4, the sequence represented by SEQ ID NO: 5, the sequence represented by SEQ ID NO: 7 or the sequence represented by SEQ ID NO: 8, or a polynucleotide consisting of a complementary sequence thereof, or
a vector capable of expressing a crRNA that binds to the polynucleotide consisting of the sequence represented by SEQ ID NO: 1, the sequence represented by SEQ ID NO: 2, the sequence represented by SEQ ID NO: 4, the sequence represented by, SEQ ID NO: 5, the sequence represented by SEQ ID NO: 7 or the sequence represented by SEQ ID NO: 8, or the polynucleotide consisting of the complementary sequence thereof.

### Item 13

A kit for preventing and/or treating heart failure comprising the composition according to item 12.

### Advantageous Effects of Invention

An object of the present invention is to provide a polynucleotide that can be inserted into an expression vector and can enhance transcriptional activity of a predetermined promoter under heart failure.

### Brief Description of Drawings

Fig. 1 shows a design of a knockout mouse (KO) in a CR9 region.
Fig. 2A shows expression levels of ANP mRNA in 4 wild-type mice and 4 KO mice. Fig. 2B shows expression levels of BNP mRNA in 4 wild-type mice and 4 KO mice.
Fig. 3A shows results of echocardiography of Sham mice and KO mice. Fig. 3B shows macroscopic findings of hearts of Sham mice and KO mice. Fig. 3C shows heart weights of Sham mice and KO mice without TAC treatment or with TAC treatment.
Fig. 4 shows a design of a reporter vector.
Fig. 5 shows designs of reporter vectors obtained by deleting a part of a CR9 region in units of 30 bp.
Fig. 6 shows luciferase activity levels of each of the deleted reporter vectors.
Fig. 7 shows enhancer active sites within a CR9 region determined from each of the deleted reporter vectors.
Fig. 8 shows a design of a reporter vector having an enhancer sequence of Example 1.
Fig. 9 shows a comparison between the reporter vector having the enhancer sequence of the Example 1 and other reporter vectors.
Fig. 10 shows an alignment between a mouse CR9 region and a human CR9 region.
Fig. 11 shows a design of the reporter vector having an enhancer sequence of Example 2.
Fig. 12 shows a comparison between the reporter vector having the enhancer sequence of the Example 2 and other reporter vectors.
Fig. 13 shows enhancer activity levels when deleting a part of the range of a mouse CR9:120 bp (191-250/311-370) in units of 10 bp. Fig. 13A shows a design of a construct used for luciferase assay. Fig. 13B shows enhancer activity levels of each construct.
Fig. 14 shows enhancer activity levels of deletion mutants obtained by deleting a part of the 318-330 region, the 331-340 region, and the 341-350 region are deleted in nits of 4 bp each.
Fig. 15 shows an alignment between a mouse 331-342 region and a human 325-336 region.
Fig. 16 shows enhancer activity levels when an enhancer sequence of a mouse 327-346 region containing the mouse 331-342 region and an enhancer sequence in which the 327-346 region was tandemly repeated three times are used. Fig. 16A shows a design of a construct used for luciferase assay. Fig. 16B shows enhancer activity levels of each construct.
Fig. 17 shows enhancer activity levels of a BNP promoter vector containing a 186-244 region and a 305-365 region (also referred to as HsCR9 120bp) in human cardiomyocytes. Fig. 17A shows a relative expression level of Nppb to an expression level of GAPDH. Fig. 17B shows a relative expression level of luciferase to the expression level of GAPDH.
Fig. 18 shows captured images of luminescence in i: AAV6_(cTnT promoter)_nFLAG MmBNP_T2A_FLuc-transferred mouse (upper) and ii: AAV6_MmCR9 120bp (cTnT promoter)_nFLAG MmBNP_T2A_FLuc-transferred mouse (lower).
Fig. 19 shows captured images of luminescence in hearts, livers, spleens, thymuses, intestines, smooth muscles, and brains excised from each mouse of the above i and ii.
Fig. 20 shows captured images of luminescence in iii: AAV6_(BNP promoter)_nFLAG MmBNP_T2A_FLuc-transferred mouse (upper) and iv: AAV6_MmCR9 120bp (BNP promoter)_nFLAG MmBNP_T2A_FLuc-transferred mouse (lower).
Fig. 21 shows captured images of luminescence in hearts, livers, spleens, thymuses, intestines, smooth muscles, and brains excised from each mouse of the above iii and iv.
Fig. 22 shows relative expression levels of luciferase mRNA to the amount of AAV6 DNA in the heart and liver removed from each mouse of the above i to iv.

### Description of Embodiments

### 1. Enhancer polynucleotide

A first embodiment relates to an enhancer polynucleotide that responds to heart failure. The enhancer is intended for a base sequence capable of enhancing transcriptional activity of a predetermined promoter. Therefore, the enhancer polynucleotide is intended for a polynucleotide that has activity of enhancing the transcriptional activity of the predetermined promoter.

Nucleotide sequences represented by each SEQ ID NO are described later.

The enhancer polynucleotide according to the first embodiment comprises a polynucleotide consisting of a sequence having 80% or more identity with a sequence represented by SEQ ID NO: 1, and includes an enhancer polynucleotide that responds to heart failure. The sequence having 80% or more identity with the sequence represented by SEQ ID NO: 1 functions as an enhancer sequence that responds to heart failure in mammals, particularly, preferably in humans.

Further, the enhancer polynucleotide according to the first embodiment comprises a polynucleotide consisting of a sequence having 80% or more identity with a sequence represented by SEQ ID NO: 2, and includes the enhancer polynucleotide that responds to heart failure. The sequence having 80% or more identity with the sequence represented by SEQ ID NO: 2 functions as an enhancer sequence that responds to heart failure in mammals, particularly, preferably in mice.

Identity with the sequence represented by SEQ ID NO: 1 or SEQ ID NO: 2 is not limited as long as it can responds to heart failure. For example, the identity is 80% or more, preferably 83% or more, and more preferably 91% or more. The length of the sequence represented by SEQ ID NO: 1 or SEQ ID NO: 2 is 12 nucleotides when expressed as a single-stranded polynucleotide. The enhancer polynucleotide may contain substitution, insertion, and/or deletion of about 1 or 2 nucleotides out of the 12 nucleotides. The substitution, insertion, and/or deletion of the nucleotide may be at any of the 12 nucleotides.

As the sequence having 80% or more identity with the sequence represented by SEQ ID NO: 1 or SEQ ID NO: 2, for example, a sequence represented by SEQ ID NO: 3 can be mentioned.

An enhancer polynucleotide comprising a polynucleotide consisting of the sequence having 80% or more identity with the sequence represented by SEQ ID NO: 1 may contain a polynucleotide consisting of a sequence having 70% or more identity with a sequence represented by SEQ ID NO: 4. The enhancer polynucleotide comprising the polynucleotide consisting of the sequence having 80% or more identity with the sequence represented by SEQ ID NO: 1 has preferably 73% or more, 78% or more, 80% or more, 83% or more, 88% or more, 90% or more, 93% or more, 95% or more, 97% or more, 98% or more, 99% or more, or 100% identity with the sequence represented by SEQ ID NO: 4. These lower limits of identity are supported in the examples described below.

An enhancer polynucleotide comprising the polynucleotide having the sequence having 80% or more identity with the sequence represented by SEQ ID NO: 2 may contain a polynucleotide consisting of a sequence having 70% or more identity with a sequence represented by SEQ ID NO: 7. The enhancer polynucleotide comprising the polynucleotide consisting of a sequence having 80% or more identity with the sequence represented by SEQ ID NO: 2 has preferably 73% or more, 78% or more, 80% or more, 83% or more, 88% or more, 90% or more, 93% or more, 95% or more, 97% or more, 98% or more, 99% or more, or 100% identity with the sequence represented by SEQ ID NO: 7. These lower limits of identity are supported in the examples described below.

In the present description, the enhancer polynucleotide comprising the polynucleotide consisting of the sequence having 80% or more identity with the sequence represented by SEQ ID NO: 1 or SEQ ID NO: 2 may be referred to as a first enhancer polynucleotide, and the sequence of the first enhancer polynucleotide may be referred to as a first enhancer sequence.

The polynucleotide consisting of the sequence having 80% or more identity with the sequence represented by SEQ ID NO: 1 or SEQ ID NO: 2 may be one unit, and the unit may be used alone, or a plurality of units may be connected to be integrated into the first enhancer polynucleotide as a tandem sequence. When using a plurality of units, same polynucleotides or different polynucleotides, selected from the polynucleotide consisting of the sequence having 80% or more identity with the sequence represented by SEQ ID NO: 1 and the polynucleotide consisting of the sequence having 80% or more identity with the sequence represented by SEQ ID NO: 2 may be combined.

Here, it is preferable that the first enhancer sequence does not comprise a polynucleotide having the same sequence as a sequence represented by SEQ ID NO: 13 or a polynucleotide having the same sequence as a sequence represented by SEQ ID NO: 12.

The length of the first enhancer polynucleotide is not limited as long as it is 10 or more nucleotides in terms of the number of nucleotides in a single-stranded polynucleotide. It is preferably 12 nucleotides or more. The length of the first enhancer polynucleotide is 100 nucleotides or less, 60 nucleotides or less, 59 nucleotides or less, 58 nucleotides or less, 50 nucleotides or less, or 40 nucleotides or less in terms of the number of nucleotides in a single-stranded polynucleotide. The upper and lower limits of the length of the enhancer polynucleotide can be combined, respectively.

The enhancer polynucleotide that responds to heart failure may be a polynucleotide comprising a second enhancer sequence. The second enhancer sequence encodes a second enhancer polynucleotide.

The second enhancer polynucleotide may, in one embodiment, comprise a polynucleotide consisting of a sequence having 90% or more identity with a sequence represented by SEQ ID NO: 5. The second enhancer polynucleotide has preferably 91% or more, 92% or more, 93% or more, 95% or more, 97% or more, 98% or more, 99% or more, or 100% identity with the sequence represented by SEQ ID NO: 5. These lower limits of identity are supported in the examples described below. In addition, it is preferable that the first to tenth sequences of the sequence represented by SEQ ID NO: 5 maintain 90% or more, or 100% identity with the second enhancer polynucleotide. The second enhancer polynucleotide preferably comprises a polynucleotide consisting of a sequence having 90% or more identity with the first to tenth sequences of the sequence represented by SEQ ID NO: 5.

The second enhancer polynucleotide may, in one embodiment, comprise a polynucleotide consisting of a sequence having 90% or more identity with a sequence represented by SEQ ID NO: 8. It is preferable that the second enhancer polynucleotide has preferably 91% or more, 92% or more, 93% or more, 95% or more, 97% or more, 98% or more, 99% or more, or 100% identity with the sequence represented by SEQ ID NO: 8. These lower limits of identity are supported in the examples described below. In addition, it is preferable that the first to tenth sequences of the sequence represented by SEQ ID NO: 8 maintain 90% or more, or 100% identity with the second enhancer polynucleotide. The second enhancer polynucleotide preferably comprises a polynucleotide consisting of a sequence having 90% or more identity with the first to tenth sequences of the sequence represented by SEQ ID NO: 8.

Here, preferably, the second enhancer sequence also does not contain the polynucleotide having the same sequence as the sequence represented by SEQ ID NO: 13, or the polynucleotide having the same sequence as the sequence represented by SEQ ID NO: 12.

The length of the second enhancer polynucleotide is not limited as long as it is 56 or more nucleotides in terms of the number of nucleotides in a single-stranded polynucleotide. It is preferably 59 nucleotides or more. The length of the second enhancer polynucleotide is 100 nucleotides or less, or 65 nucleotides or less in terms of the number of nucleotides in a single-stranded polynucleotide. The upper and lower limits of the length of the enhancer polynucleotide can be combined, respectively.

Furthermore, as another embodiment, the enhancer polynucleotide that responds to heart failure can preferably comprise (i) the first enhancer polynucleotide and (ii) the second enhancer polynucleotide.

When the enhancer polynucleotide responding to heart failure comprises both the second enhancer polynucleotide and the first enhancer polynucleotide, the second enhancer polynucleotide and the first enhancer polynucleotide can be combined directly or via an adapter.

Either the first enhancer polynucleotide or the second enhancer polynucleotide may be upstream, but it is preferable that the second enhancer polynucleotide is upstream of the first enhancer polynucleotide. However, when the second enhancer polynucleotide is connected with the first enhancer polynucleotide via an adapter, it is preferable that the polynucleotide having the same sequence as the sequence represented by SEQ ID NO: 13 or the polynucleotide having the same sequence as the sequence represented by SEQ ID NO: 12 is not comprised. In particular, when the first enhancer polynucleotide is connected to the downstream side of the second enhancer polynucleotide, it is preferable not to include an embodiment in which a polynucleotide consisting of a sequence having 95% or more identity with a sequence represented by SEQ ID NO: 6 or a polynucleotide consisting of a sequence having 95% or more identity with a sequence represented by SEQ ID NO: 9 is connected to the second enhancer polynucleotide at the position directly below it, and further, the first enhancer polynucleotide is connected to the polynucleotide at the position directly below it.

When the first enhancer polynucleotide comprises the polynucleotide consisting of the sequence having 80% or more identity with the sequence represented by SEQ ID NO: 1, it is preferable that the second enhancer polynucleotide comprises the polynucleotide consisting of the sequence having 90% or more identity with the sequence represented by SEQ ID NO: 5. In this case, it is preferable that an entire enhancer polynucleotide responding to heart failure is not a polynucleotide having the same sequence as the sequence represented by SEQ ID NO: 13. In other words, an embodiment in which a second enhancer polynucleotide consisting of a sequence having 100% identity with the sequence represented by SEQ ID NO: 5, a polynucleotide represented by SEQ ID NO: 6, and a first enhancer polynucleotide consisting of a sequence having 100% identity with the sequence represented by SEQ ID NO: 4 are directly combined in order from 5' terminal side is excluded from the enhancer polynucleotide responding to heart failure.

When the first enhancer polynucleotide comprises the polynucleotide consisting of the sequence having 80% or more identity with the sequence represented by SEQ ID NO: 2, it is preferable that the second enhancer polynucleotide comprises the polynucleotide consisting of the sequence having 90% or more identity with the sequence represented by SEQ ID NO: 8. In this case, it is preferable that an entire enhancer polynucleotide responding to heart failure is not a polynucleotide having the same sequence as the sequence represented by SEQ ID NO: 12. In other words, an embodiment in which a second enhancer polynucleotide consisting of a sequence having 100% identity with the sequence represented by SEQ ID NO: 8, a polynucleotide represented by SEQ ID NO: 9, and a first enhancer polynucleotide consisting of a sequence having 100% identity with the sequence represented by SEQ ID NO: 7 are directly combined in order from 5' terminal side is excluded from the enhancer polynucleotide responding to heart failure.

When an adapter is included between the second enhancer polynucleotide and the first enhancer polynucleotide, the length of the adapter is preferably 80 nucleotides or less, 60 nucleotides or less, 40 nucleotides or less, 30 nucleotides or less, 20 nucleotides or less, 10 nucleotides or less, 8 nucleotides or less, 6 nucleotides or less, or 5 nucleotides or less in terms of the number of nucleotides in a single strand.

The enhancer polynucleotide responding to heart failure may be single strand or double strand. When the enhancer polynucleotide responding to heart failure is single strand, it may have the same sequence as the sequence of the enhancer polynucleotide responding to heart failure as described above, and may have an inverted complementary sequence to the sequence of the enhancer polynucleotide responding to heart failure as described above. Preferably, the enhancer polynucleotide responding to heart failure is double strand.

The total length of the enhancer polynucleotide responding to heart failure is preferably 200 nucleotides or less, 150 nucleotides or less, 130 nucleotides or less, 125 nucleotides or less, or 120 nucleotides or less when the length of the enhancer polynucleotide is expressed by the number of nucleotides in a single strand. When the enhancer polynucleotide responding to heart failure includes an adapter, the length of the enhancer polynucleotide responding to heart failure indicates the number of nucleotides including the adapter.

The enhancer polynucleotide responding to heart failure is to be used in combination with polynucleotides comprising promoter sequences and polynucleotides to be expressed, as described below. However, there are cases where the length of the polynucleotide that can be integrated into the viral vector is limited. Therefore, it is preferable that the enhancer polynucleotide is as short as possible.

The most preferred enhancer polynucleotide responding to heart failure is the polynucleotide having the sequence represented by SEQ ID NO: 10 or the polynucleotide having the sequence represented by SEQ ID NO: 11.

Activity as an enhancer responding to heart failure (hereinafter simply referred to as "enhancer activity") can be assessed by reporter assay. For example, The enhancer activity can be evaluated by comparing the reporter activity in a cell transfected with a vector for reporter assay to which a reporter gene such as luciferase or β-galactosidase is integrated in a downstream region of a predetermined promoter sequence with the reporter activity in a cell transfected with an enhancer-added vector for reporter assay to which an enhancer sequence is inserted in an upstream region or a downstream region of the promoter sequence of the same vector with the vector for reporter assay. With the reporter activity in the cell transfected with a vector for reporter assay being set as a baseline (also referred to as the reference value), when the reporter activity in a cell transfected with the enhancer-added vector for reporter assay (also referred to as the evaluation value) is higher than the baseline, it can be determined that the enhancer polynucleotide has enhancer activity. More specifically, when the evaluation value is 1.5 times or more, preferably 2 times or more, more preferably 2.5 times or more, and further preferably 3 times or more higher than the reference value, it can be determined that the enhancer polynucleotide has enhancer activity.

Whether or not the enhancer polynucleotide can respond to heart failure can be evaluated by exposing cardiomyocytes into which the enhancer-added reporter assay vector has been introduced to a pseudo heart failure state environment in vitro. For example, the vector is introduced into myocardial cells cultured in two different wells, and an adrenaline α₁ receptor agonist or the like such as phenylephrine is added to one of the wells to expose the gene-introduced myocardial cells to the pseudo heart failure state environment. The responsivity of the enhancer polynucleotide to heart failure can be evaluated by comparing reporter activity when exposed to the pseudo heart failure state environment (also referred to as treated evaluation value) with reporter activity when not exposed to the pseudo heart failure state environment (also referred to as untreated evaluation value). When the treated evaluation value is 2 times or more, preferably 3 times or more, more preferably 4 times or more higher than the untreated evaluation value, it can be evaluated that the enhancer is responsive to heart failure.

Here the reporter assay can be performed according to known methods.

The predetermined promoter is not limited as long as the gene to be expressed can be expressed in the gene-transfected cell. As the predetermined promoter, promoters of the adjacent genes Nppa and Nppb encoding ANP and BNP, which are endogenous target promoters of the enhancer sequence (for example, a promoter consisting of the sequence represented by SEQ ID NO: 14 below), can be used. It is also possible to use an exogenous promoter as the predetermined promoter. As exogenous promoters, cytomegalovirus (CMV) promoter, Respiratory syncytial virus (RSV) promoter, Simian virus 40 (SV40) promoter, Elongation factor 1 (EF1) -α promoter, Troponin T (TnT) promoter, etc. can be included. These promoters are preferably minimal promoters that do not have the enhancers associated with these promoters.

It is shown in Non Patent Literature 2 that the transcriptional activity of the exogenous promoter can be enhanced by the CR9 region (650 bp) of the adjacent genes Nppa and Nppb.

### 2. Expression vector and composition for gene therapy

A third embodiment relates to an expression vector and a composition for gene therapy. The expression vector in this embodiment can comprise enhancer polynucleotide responding to heart failure described in the above 1, and the polynucleotide comprising the predetermined promoter sequence described in the above 1. In the expression vector, the enhancer polynucleotide responding to heart failure and the polynucleotide comprising the promoter sequence can be connected so that the transcription activity of the polynucleotide comprising the promoter sequence is enhanced by the enhancer polynucleotide responding to heart failure.

The enhancer polynucleotide responding to heart failure is not limited as long as the polynucleotide comprising the promoter sequence is connected so as to be able to function. The enhancer polynucleotide responding to heart failure may be positioned in the 5' side upstream region of the polynucleotide comprising the promoter sequence, or may be positioned in the 3' side downstream region of the polynucleotide comprising the promoter sequence.

Further, the expression vector may comprise a polynucleotide to be expressed. The polynucleotide to be expressed may have a sequence encoding a protein, microRNA, siRNA, or the like. The polynucleotide to be expressed is preferably inserted into the expression vector so that the polynucleotide comprising the promoter sequence and the enhancer polynucleotide express the sequence encoded by the polynucleotide to be expressed.

Basic skeleton of the expression vector is not limited as long as the polynucleotide to be expressed can be expressed by the polynucleotide comprising the promoter sequence and the enhancer polynucleotide, in mammalian cells. Examples of the basic skeleton of the expression vector include a plasmid vector, a virus vector and the like. Examples of the virus vector can include a lentivirus vector, a retrovirus vector, an adenovirus vector, an adeno-associated virus vector (AAV vector) and the like. In the case of AAV vector, a serotype of the AAV vector may be any of AAV1, AAV2, AAV3, AAV4, AAV5, AAV6, AAV7, AAV8, AAV9, AAV10 and AAV11, but when infecting cardiomyocytes, any of AAV1, AAV3, AAV4, AAV6 and AAV9 is preferably used.

The expression vector of this embodiment can be used as an active ingredient of a composition for gene therapy. In particular, it can be used as an active ingredient in a composition for gene therapy of heart disease, preferably heart failure.

In addition to the expression vector, the composition for gene therapy may comprise a carrier, if necessary. Examples of the carrier may include transfection reagents comprising polymers, lipids, magnetism and the like.

When the composition for gene therapy is administered to an individual, it can be administered systemically or locally. When it is administered systemically, intravenous injection is preferred.

When basic skeleton of the active ingredient is a virus vector and the composition for gene therapy is systemically administered, for example, it can be administered so as to be 10¹⁰ to 10¹⁸ vg/day per 1 kg of adult body weight in terms of the amount of the active ingredient.

When the basic skeleton of the active ingredient is the virus vector and the composition for gene therapy is locally administered, the composition for gene therapy can be injected into target tissue using a syringe or a catheter. In the case of local administration, for example, it can be administered so as to be 10⁹ to 10¹⁶ vg/day per 1 cm² of the target tissue in terms of the amount of the active ingredient.

When the basic skeleton of the active ingredient is the virus vector, the composition for gene therapy can be administered once or a plurality of times regardless of whether it is systemically administered or locally administered. When it is administered a plurality of times, the administration can be repeated every 2 days, 4 days, or 1 week. When it is administered a plurality of times, it can be administered 2 times, 5 times, 10 times, 15 times, 20 times or 24 times.

When basic skeleton of the active ingredient is a plasmid vector and the composition for gene therapy is systemically administered, it can be administered, for example, so as to be 0.1 to 1,000 mg/day per 1 kg of adult body weight in terms of the amount of the active ingredient. The vector can be linearized if necessary.

When basic skeleton of the active ingredient is a plasmid vector and the composition for gene therapy is locally administered, the composition for gene therapy can be injected into a target tissue using a syringe or a catheter. In this case, a nucleic acid delivery reagent such as liposome and the like may be used in combination. In the case of local administration, for example, it can be administered so as to be 0.01 to 100 mg/day per 1 cm² of the target tissue in terms of the amount of the active ingredient.

When basic skeleton of the active ingredient is a plasmid vector, the composition for gene therapy can be administered once or a plurality of times regardless of whether it is systemically administered or locally administered. When it is administered a plurality of times, the administration can be repeated every 2 days, 4 days or 1 week. When it is administered a plurality of times, it can be administered 2 times, 5 times, 10 times, 15 times, 20 times or 24 times.

The composition for gene therapy can be used for prevention or treatment of heart failure. Here, "prevention" is intended to prevent onset or recurrence. "Treatment" includes ameliorating heart failure, and maintaining heart failure, if any, without exacerbation.

Myosin light chain kinase 3 (Mylk3) cDNA, ANP cDNA, BNP cDNA and the like, which have a protective effect on cardiac function, can be transferred to the expression vector for preventing or treating heart failure as the polynucleotide to be expressed.

### 3. Composition and kit comprising sgRNA or crRNA

A fourth embodiment relates to a composition for preventing or treating heart failure comprising a single-stranded guide RNA (sgRNA) or a vector capable of expressing the sgRNA as an active ingredient. The composition can be used for activation of an endogenous enhancer region.

The sgRNA can be designed to bind to the polynucleotide consisting of the sequence represented by SEQ ID NO: 1, the sequence represented by SEQ ID NO: 2, the sequence represented by SEQ ID NO: 4, the sequence represented by SEQ ID NO: 5, the sequence represented by SEQ ID NO: 7 or the sequence represented by SEQ ID NO: 8 or a polynucleotide consisting of a complementary sequence thereof. Candidate sequences provided by each manufacturer can be used as the sequence of the sgRNA.

The sgRNA or the vector capable of expressing the sgRNA can enhance the expression of the endogenous gene of interest by being transferred to a cell together with a vector expressing a tetrameric herpes virus transcription activation domain VP16 (VP64) which is transactivation domain and a vector expressing an inactive Cas9 nuclease. Preferably, the VP64 and the inactive Cas9 nuclease can be fused and expressed intracellularly. The inactive Cas9 nuclease preferably has a D10A mutation in which the 10th aspartic acid is substituted with alanine and/or an N863A mutation in which the 863th asparagine is substituted with alanine. In addition, when transferring sgRNA or the vector capable of expressing the sgRNA, and the vector expressing VP64 and the inactive Cas9 nuclease, it is preferable to transfer a vector expressing p65. Further, Transcription Factor p65 (p65) is preferably expressed intracellularly together with MS2 coat protein (MS2) and Heat Shock Transcription Factor 1 (HSF1), and is more preferably expressed intracellularly, for example, as an MS2-p65-HSF1 fusion protein. The inactive Cas9 nuclease may be expressed intracellularly with VP64 and p65, along with Replication and transcription activator (Rta).

As the basic skeletons of the vector capable of expressing the sgRNA, the vector expressing VP64 and the inactive Cas9 nuclease, and the vector expressing p65, MS2 and HSF1, the basic skeletons described in the above 2. are included.

The basic skeletons of the vector capable of expressing the sgRNA, the vector expressing VP64 and the inactive Cas9 nuclease, and the vector expressing p65, MS2 and HSF1 can be administered to an individual, for example, in the same number of copies.

The method of introducing each vector can be performed in accordance with the description in the above 2.

When the sgRNA is systemically administered, it can be administered, for example, so as to be 0.1 to 1,000 mg/day per 1 kg of adult body weight in terms of the amount of the active ingredient. The vector can be linearized if necessary.

When the sgRNA is locally administered, the composition for gene therapy can be injected into the target tissue using a syringe or a catheter. In this case, a nucleic acid delivery reagent such as liposome and the like may be used in combination. In the case of local administration, for example, it can be administered so as to be 0.01 to 100 mg/day per 1 cm² of the target tissue in terms of the amount of the active ingredient.

The sgRNA and each vector can be administered once or a plurality of times regardless of whether it is systemically administered or locally administered. When it is administered a plurality of times, the administration can be repeated every 2 days, 4 days or 1 week. When it is administered a plurality of times, it can be administered 2 times, 5 times, 10 times, 15 times, 20 times or 24 times.

In addition, CRISPR RNA (crRNA) or a vector capable of expressing crRNA may be used instead of the sgRNA or the vector capable of expressing the sgRNA. The crRNA can be administered systemically or locally, preferably in a state of being hybridized with tracrRNA. The vector capable of expressing crRNA preferably expresses tracrRNA together with crRNA, or is preferably combined with a vector expressing tracrRNA. When using crRNA, Rta may be used instead of HSF1. The crRNA is also designed to bind to the polynucleotide consisting of the sequence represented by SEQ ID NO: 1, the sequence represented by SEQ ID NO: 2, the sequence represented by SEQ ID NO: 4, the sequence represented by SEQ ID NO: 5, the sequence represented by SEQ ID NO: 7, or the sequence represented by SEQ ID NO: 8, or the polynucleotide consisting of the complementary sequence thereof. A candidate sequence provided by each manufacturer can be used as a sequence of crRNA.

The composition comprises a nucleic acid such as a sgRNA, crRNA and tracrRNA, the expression vector expressing a sgRNA or crRNA and the like as an active ingredient, and may contain a carrier if necessary. Examples of the carrier can include transfection reagent comprising polymer, lipid, magnetism and the like.

The composition can be used to prevent and/or treat heart failure. In addition, the composition may be provided as a kit in combination with, for example, an expression vector expressing the fusion protein of VP64 and an inactive Cas9 nuclease, and the MS2-p65-HSF1 fusion protein expression vector. Further, the composition may be provided as a kit in combination with an expression vector expressing an inactive Cas9 nuclease-VP64-p65-Rta fusion protein. The kit can be used for prevention or treatment of heart failure. Here, "prevention" is intended to prevent onset or recurrence. "Treatment" includes ameliorating heart failure, and maintaining heart failure, if any, without exacerbation.

### 4. Sequence listing

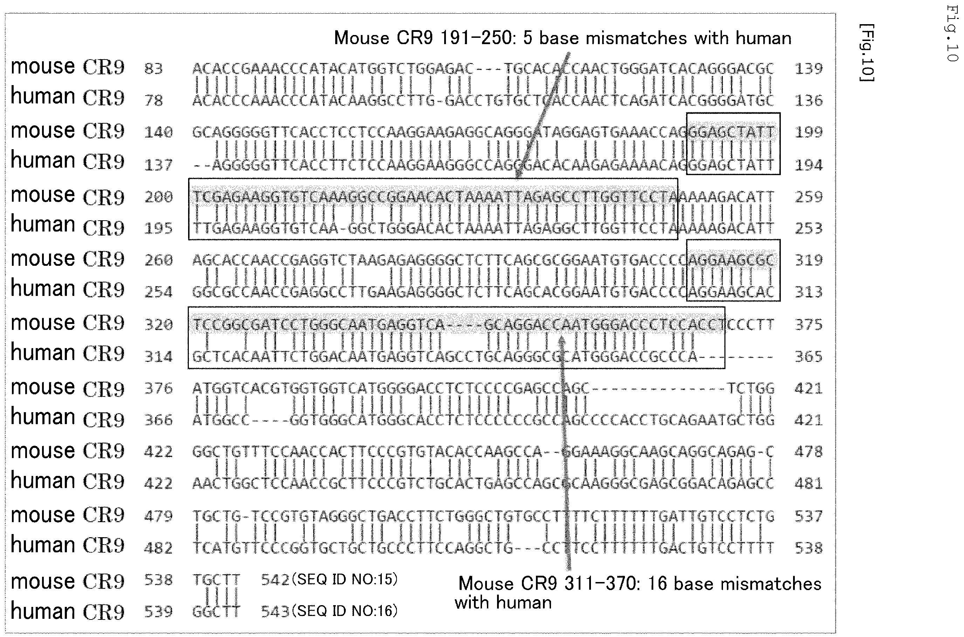

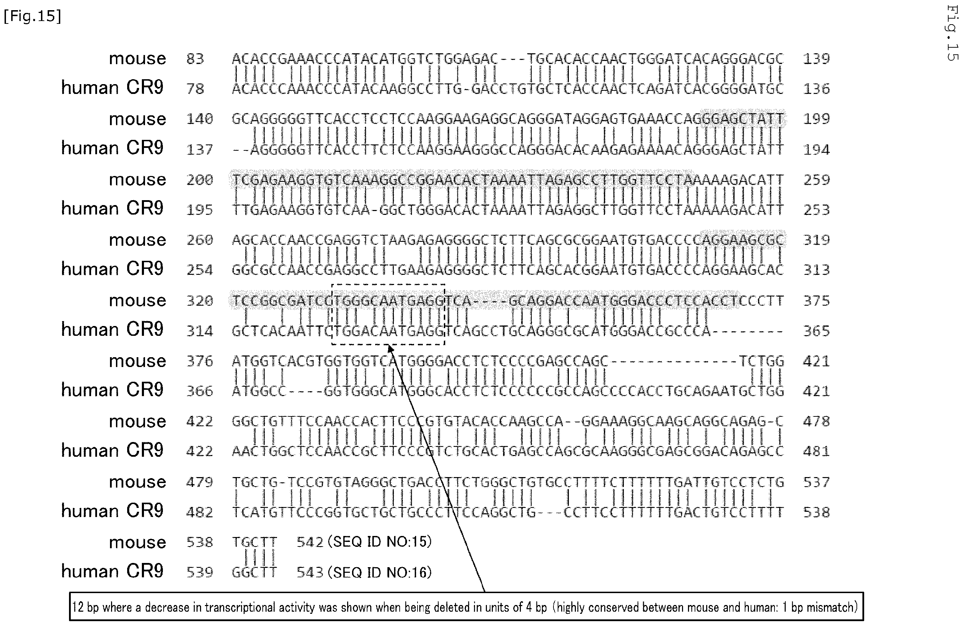

The sequences of SEQ ID NOs: 1 to 14 disclosed in the present specification are shown below.
- SEQ ID NO: 1 (human 325-336)
   TGGACAATGAGG
- SEQ ID NO: 2 (mouse 331-342)
   TGGGCAATGAGG
- SEQ ID NO: 3 (general sequence)
   TGGNCAATGAGG
   (N is A, G, T, or C.)
- SEQ ID NO: 4 (human 305-365) 
AGGAAGCACGCTCACAATTCTGGACAATGAGGTCAGCCTGCAGGGCGCATGGGACC
GCCCA
 (The underlined part indicates the part corresponding to SEQ ID NO: 1.)
- SEQ ID NO: 5 (human 186-244) 
GGAGCTATTTGAGAAGGTGTCAAGGCTGGGACACTAAAATTAGAGGCTTGGTTCCT
AA
- SEQ ID NO: 6 (human 245-304) 
AAAGACATTGGCGCCAACCGAGGCCTTGAAGAGGGGCTCTTCAGCACGGAATGTGA
CCCC
- SEQ ID NO: 7 (mouse 311-370) 
AGGAAGCGCTCCGGCGATCCTGGGCAATGAGGTCAGCAGGACCAATGGGACCCTCC
ACCT
 (The underlined part indicates the part corresponding to SEQ ID NO: 2.)
- SEQ ID NO: 8 (mouse 191-250) 
GGAGCTATTTCGAGAAGGTGTCAAAGGCCGGAACACTAAAATTAGAGCCTTGGTTC
CTAA
- SEQ ID NO: 9 (mouse 251-310) 
AAAGACATTAGCACCAACCGAGGTCTAAGAGAGGGGCTCTTCAGCGCGGAATGTGA
CCCC
- SEQ ID NO: 10 (Example 2: human) 
GGAGCTATTTGAGAAGGTGTCAAGGCTGGGACACTAAAATTAGAGGCTTGGTTCCT
AAGAATTCAGGAAGCACGCTCACAATTCTGGACAATGAGGTCAGCCTGCAGGGCGC
ATGGGACCGCCCA
- SEQ ID NO: 11 (Example 1: mouse) 
GGAGCTATTTCGAGAAGGTGTCAAAGGCCGGAACACTAAAATTAGAGCCTTGGTTC
CTAAACTAGAGGAAGCGCTCCGGCGATCCTGGGCAATGAGGTCAGCAGGACCAATG
GGACCCTCCACCT
- SEQ ID NO: 12 (mouse CR9) 
CTCATGGTGCCAGTTGCTGCCCTTGGCCAAAAACATGTCCTCTTCATCCAGCTTCATA
AACACGGCAGGGACAGAACTTGCCACACCGAAACCCATACATGGTCTGGAGACTGC
ACACCAACTGGGATCACAGGGACGCGCAGGGGGTTCACCTCCTCCAAGGAAGAGGC
AGGGATAGGAGTGAAACCAGGGAGCTATTTCGAGAAGGTGTCAAAGGCCGGAACA
CTAAAATTAGAGCCTTGGTTCCTAAAAAGACATTAGCACCAACCGAGGTCTAAGAG
AGGGGCTCTTCAGCGCGGAATGTGACCCCAGGAAGCGCTCCGGCGATCCTGGGCAA
TGAGGTCAGCAGGACCAATGGGACCCTCCACCTCCCTTATGGTCACGTGGTGGTCAT
GGGGACCTCTCCCCGAGCCAGCTCTGGGGCTGTTTCCAACCACTTCCCGTGTACACC
AAGCCAGGAAAGGCAAGCAGGCAGAGCTGCTGTCCGTGTAGGGCTGACCTTCTGGG
CTGTGCCTTTTCTTTTTTGATTGTCCTCTGTGCTTTCTGTTCCATTCTCAGTTTAAGAG
TGGGAGAGGAGGATGCTTGGAAGAGGCAGCCATCTTGGAACCTGGTGGTTTGGCCT
GGGAGATAAACCAGGAAGGGCCTAGGAT
- SEQ ID NO: 13 (human CR9) 
GTGCTGTGAGCTGCCCTCCCAGAGGTGAGTTCTCTGGGTCCTGCCACACAAAACACA
TGGCAGGGGCGTGGATCCCTACACCCAAACCCATACAAGGCCTTGGACCTGTGCTC
ACCAACTCAGATCACGGGATGCAGGGGGTTCACCTTCTCCAAGGAAGGCCAGGG
ACACAAGAGAAACAGGGAGCTATTTGAGAAGGTGTCAAGGCTGGGACACTAAA
ATTAGAGGCTTGGTTCCTAAAAGACATTGGCGCCAACCGAGGCCTTGAAGAGGGG
CTCTTCAGCACGGAATGTGACCCCAGGAAGCACGCTCACAATTCTGGACAATGAGG
 
TCAGCCTGCAGGGCGCATGGGACCGCCCAATGGCCGGTGGGCATGGGCACCTCTCC
CCCCGCCAGCCCACCTGCAGAATGCTGGAACTGGCTCCAACCGCTTCCCGTCTGCA
CTGAGCCAGCGCAAGGGCGAGCGGACAGAGCCTCATGTTCCCGGTGCTGCTGCCCT
TCCAGGCTGCCTTCCTTTTTGACTGTCCTTTTGGCTTCTTATGCTTTTGGCTCCATTC
TGGATGTGCGTCATGGGGTGGATGCAGAAGCAGCAGCCAGCTTGGAGGCTGGTGT
TCCCGGAGGCTGAGGGCGGATGGGGGAAATATGAACCAGGAAAGGCCTGGTA
T
- SEQ ID NO: 14 (promoter sequence of mouse BNP) 
CCTGAGCTCAGCCGGCAGGAATGCAGCTGATAAATCAGAGATAACCCCACCCCTAC
TCCGTGAAAAGGTCTGGCCGGACACTCAGCCCCAGTATAAAGGCAGAGGCACCGT
TGTTGAAGACACCAGTGCACAAGCTGCTTGGGGAGGCGAGACAAGGGAGAACACG
GCATCATTGCCTGGCCCATCGCTTCTGCGGC
- SEQ ID NO: 15 (Figs. 10 and 15 mouse) 
ACACCGAAACCCATACATGGTCTGGAGACTGCACACCAACTGGGATCACAGGGACG
CGCAGGGGGTTCACCTCCTCCAAGGAAGAGGCAGGGATAGGAGTGAAACCAGGGA
GCTATTTCGAGAAGGTGTCAAAGGCCGGAACACTAAAATTAGAGCCTTGGTTCCTA
AAAAGACATTAGCACCAACCGAGGTCTAAGAGAGGGGCTCTTCAGCGCGGAATGTG
ACCCCAGGAAGCGCTCCGGCGATCCTGGGCAATGAGGTCAGCAGGACCAATGGGAC
CCTCCACCTCCCTTATGGTCACGTGGTGGTCATGGGGACCTCTCCCCGAGCCAGCTC
TGGGGCTGTTTCCAACCACTTCCCGTGTACACCAAGCCAGGAAAGGCAAGCAGGCA
GAGCTGCTGTCCGTGTAGGGCTGACCTTCTGGGCTGTGCCTTTTCTTTTTTGATTGTC
CTCTGTGCTT
- SEQ ID NO: 16 (Figs. 10 and 15 human)

ACACCCAAACCCATACAAGGCCTTGGACCTGTGCTCACCAACTCAGATCACGGGGA
TGCAGGGGGTTCACCTTCTCCAAGGAAGGGCCAGGGACACAAGAGAAAACAGGGA
GCTATTTTGAGAAGGTGTCAAGGCTGGGACACTAAAATTAGAGGCTTGGTTCCTAAA
AAGACATTGGCGCCAACCGAGGCCTTGAAGAGGGGCTCTTCAGCACGGAATGTGAC
CCCAGGAAGCACGCTCACAATTCTGGACAATGAGGTCAGCCTGCAGGGCGCATGGG
ACCGCCCAATGGCCGGTGGGCATGGGCACCTCTCCCCCGCCAGCCCCACCTGCAG
AATGCTGGAACTGGCTCCAACCGCTTCCCGTCTGCACTGAGCCAGCGCAAGGGCGA

GCGGACAGAGCCTCATGTTCCCGGTGCTGCTGCCCTTCCAGGCTGCCTTCCTTTTTG
ACTGTCCTTTTGGCTT

### Examples

Hereinafter, the present invention will be described in more detail with reference to examples. However, the interpretation of the present invention shall not be limited to the examples.

All animal experiments in the examples were carried out with the approval of the Animal Experiments Committee of Osaka University in accordance with the Guide for the Care and Use of Laboratory Animals.

### I. Experimental example 1: Importance of CR9 region in heart failure

A knockout mouse (KO mouse) of the CR9 region (SEQ ID NO: 12) which is one of the enhancers of the mouse natriuretic peptide (ANP/BNP) gene represented by SEQ ID NO: 12 was prepared to investigate the importance of the CR9 region under heart failure.

### 1. Preparation of CR9 650bp KO mouse

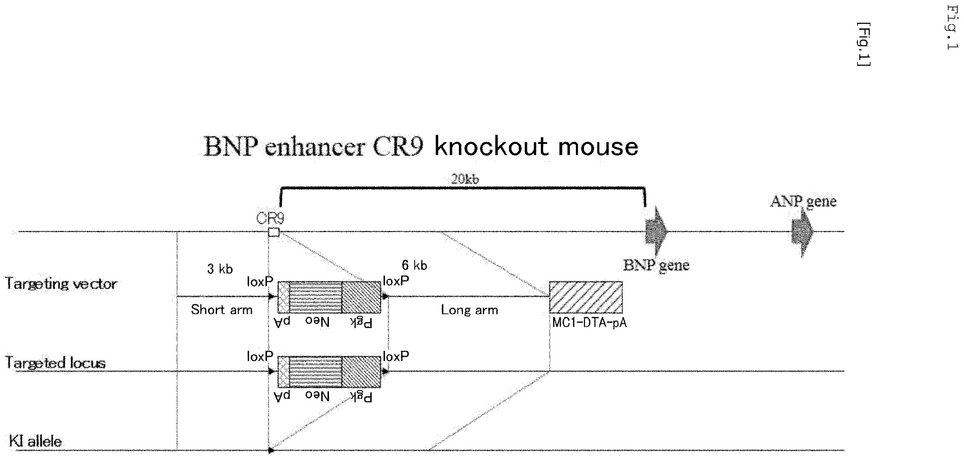

The CR9 KO mice were prepared by using Cre-LoxP system. A design of a targeting vector for CR9 KO mice is shown in the Fig. 1. The preparation of the CR9 KO mice was outsourced to the Reproductive Engineering Unit, Institutes for Animal Experimentation Affiliated with the Faculty of Medicine, Osaka University.

In order to delete the LoxP region, LoxP mice was mated with Cre-expressing mice.

### 2. Phenotype of CR9 KO mouse

### 2-1. Expression of ANP and BNP

In order to observe the expression of ANP and BNP in CR9 KO mice, hearts were excised from 4 wild-type (WT) mice and 4 CR9 KO mice which are 8-week-old, and RNA was extracted from the ventricles. RNA-Bee ^{™} was used to extract RNA.

Expression of ANP and BNP was evaluated by quantitative PCR. Beta-actin (Actb) was used as an endogenous control. SYBR ^{™} Green Realtime PCR Master Mix was used for quantitative PCR.

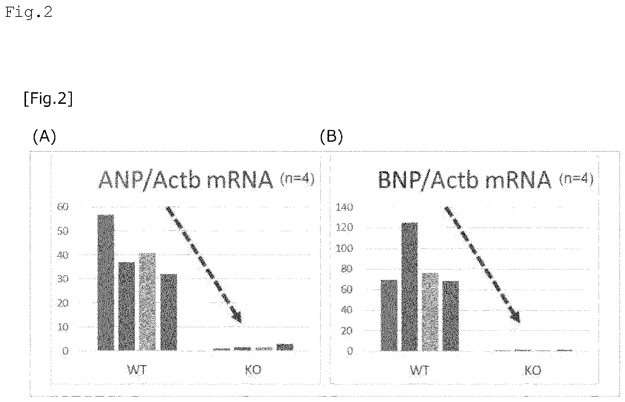

The results are shown in Fig. 2. Expression of both ANP and BNP was markedly reduced in CR9 KO mice.

### 2-2. Phenotype of CR9 KO mice in heart failure model

In order to evaluate the importance of the CR9 region under heart failure, a pressure-loaded heart failure model was prepared and the cardiac conditions of CR9 KO mice and WT mice were observed.

Transverse aortic coarctation (TAC) was performed on 4 WT mice and 4 CR9 KO mice which are 8-week-old. An outline of the TAC is as follows. After entering the thoracic cavity from the second intercostal space of the left sternal border and exposing the aortic arch, the aortic arch was tied with a 7-0 suture with a 27G needle between the innominate artery and the left internal carotid artery. The 27G needle was then carefully removed to create a 60-80% stenosis of the aorta. On the other hand, sham operation was performed on another 4 WT mice and 4 CR9 KO mice which are 8-week-old. Echocardiography and measurement of heart weight were performed 5 weeks after the operation.

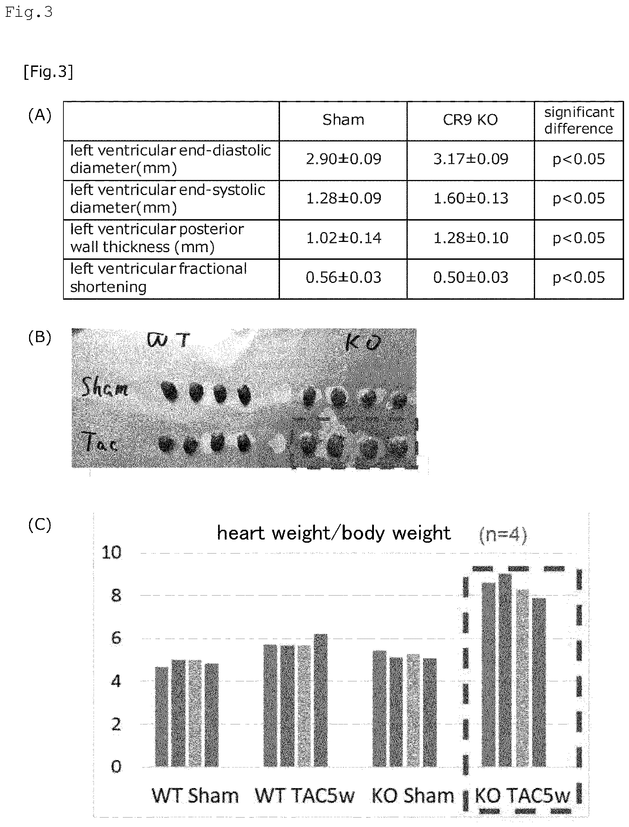

Fig. 3A shows the results of echocardiography. In CR9 KO mice, left ventricular end-diastolic diameter and left ventricular end-systolic diameter were expanded more and left ventricular posterior wall thickness was thicker than in WT mice. Left ventricular fractional shortening was significantly lower in CR9 KO mice than in WT mice. From this, it was found that CR9 KO mice cause significant cardiac enlargement, cardiac hypertrophy, and decreased left ventricular wall motion under heart failure.

Fig. 3B shows macroscopic findings of hearts excised from CR9 KO mice and WT mice that underwent TAC, and CR9 KO mice and WT mice that underwent Sham operation. Fig. 3C is a graph showing the weight of each heart as heart weight/body weight ratio. The hearts of CR9 KO mice that underwent TAC were larger than those of the other groups by macroscopic findings. In addition, the heart weight was also increased in CR9 KO mice that underwent TAC.

From the above results, it was found that the deletion of the CR9 region exacerbates heart failure. From this, it was considered that the CR9 region is essential for suppressing the exacerbation of heart failure.

### II. Experimental example 2: Analysis of a region responding to heart failure within the CR9 region 1. Search for the region responding to heart failure within the CR9 region

The mouse CR9 region is a 650 bp region represented by the following sequence.

CTCATGGTGCCAGTTGCTGCCCTTGGCCAAAAACATGTCCTCTTCATCCAGCTTCATA
AACACGGCAGGGACAGAACTTGCCACACCGAAACCCATACATGGTCTGGAGACTGC
ACACCAACTGGGATCACAGGGACGCGCAGGGGGTTCACCTCCTCCAAGGAAGAGGC
AGGGATAGGAGTGAAACCAGGGAGCTATTTCGAGAAGGTGTCAAAGGCCGGAACA
CTAAAATTAGAGCCTTGGTTCCTAAAAAGACATTAGCACCAACCGAGGTCTAAGAG
AGGGGCTCTTCAGCGCGGAATGTGACCCCAGGAAGCGCTCCGGCGATCCTGGGCAA
TGAGGTCAGCAGGACCAATGGGACCCTCCACCTCCCTTATGGTCACGTGGTGGTCAT
GGGGACCTCTCCCCGAGCCAGCTCTGGGGCTGTTTCCAACCACTTCCCGTGTACACC
AAGCCAGGAAAGGCAAGCAGGCAGAGCTGCTGTCCGTGTAGGGCTGACCTTCTGGG
CTGTGCCTTTTCTTTTTGATTGTCCTCTGTGCTTTCTGTTCCATTCTCAGTTTAAGAG
TGGGAGAGGAGGATGCTTGGAAGAGGCAGCCATCTTGGAACCTGGTGGTTTGGCCT
GGGAGATAAACCAGGAAGGGCCTAGGAT（SEQ ID NO: 12）

In order to identify a more important region in the above sequence, a vector having an enhancer region obtained by deleting a part of the 650 bp in units of 30 bp was prepared, and its transcriptional activity was evaluated.

### 1-1. Preparation of a vector

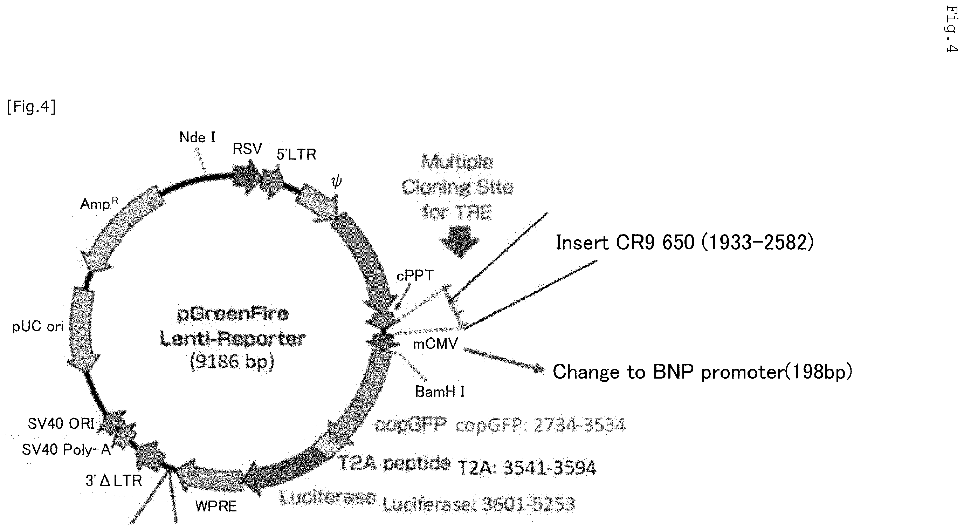

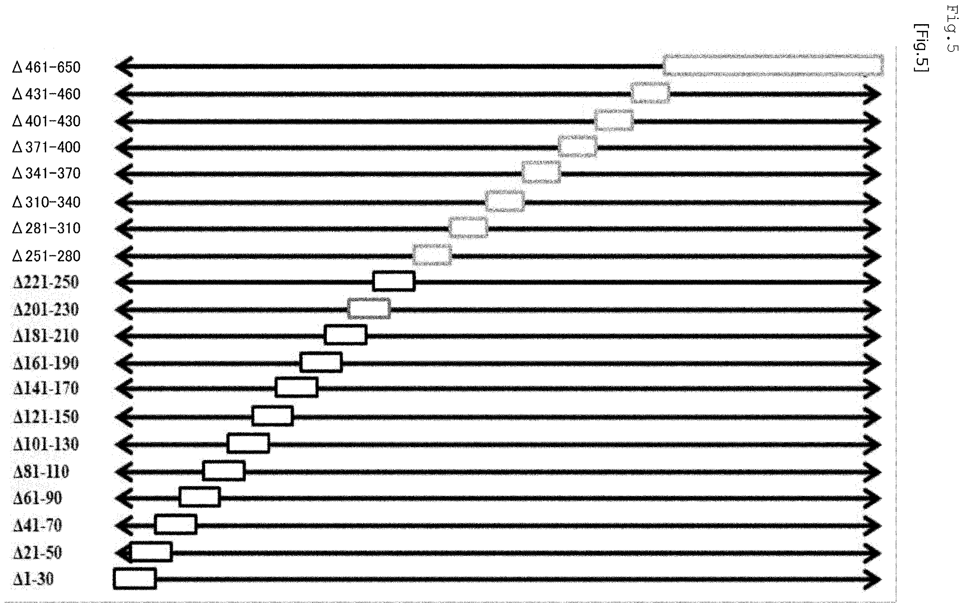

The mCMV promoter of pGreenFire Lenti-Reporter shown in Fig. 4 was replaced with the BNP promoter (198 bp) represented by SEQ ID NO: 14. The CR9 650bp was inserted into the EcoRI/SpeI site of the multi-cloning site located upstream of the BNP promoter to prepare CR9 + BNP promoter + luciferase Lentivector (hereinafter referred to as "original vector"). Based on the original vector, a Deletion Lentivector was prepared by deleting a part of a CR9 region in units of 30 bp. Fig. 5 shows the Deletion Lentivector prepared.

### 1-2. Preparation of cardiomyocytes of a rat and luciferase assay

Hearts were excised from 1-day-old rats and heart tissue was treated overnight with a trypsin solution cooled to 4°C. Then, the heart tissue was transferred to collagenase solution and incubated at 37°C. Cells were collected from digested tissue and the cells were transferred to plates with medium containing 10% fetal bovine serum and allowed to stand for 70 minutes. This treatment allows fibroblasts to adhere to the bottom of the plate and allows cardiomyocytes to remain in a supernatant. The supernatant was collected and the cardiomyocytes in the supernatant were plated on a 96-well plate.

After culturing the cardiomyocytes of the rat in a CO₂ incubator at 37°C for 24 hours, cardiomyocytes in another well were infected with each Deletion Lentivector having the deletion shown in Fig. 5. On the other hand, the cardiomyocytes were also infected with the original vector as a control. After 48 hours, Phenylephrine (Sigma-Aldrich) was added to each well to a final concentration of 100 µM, and the cells were cultured for 72 hours. After the culture was completed, luciferase assay was performed.

### 1-3. Results

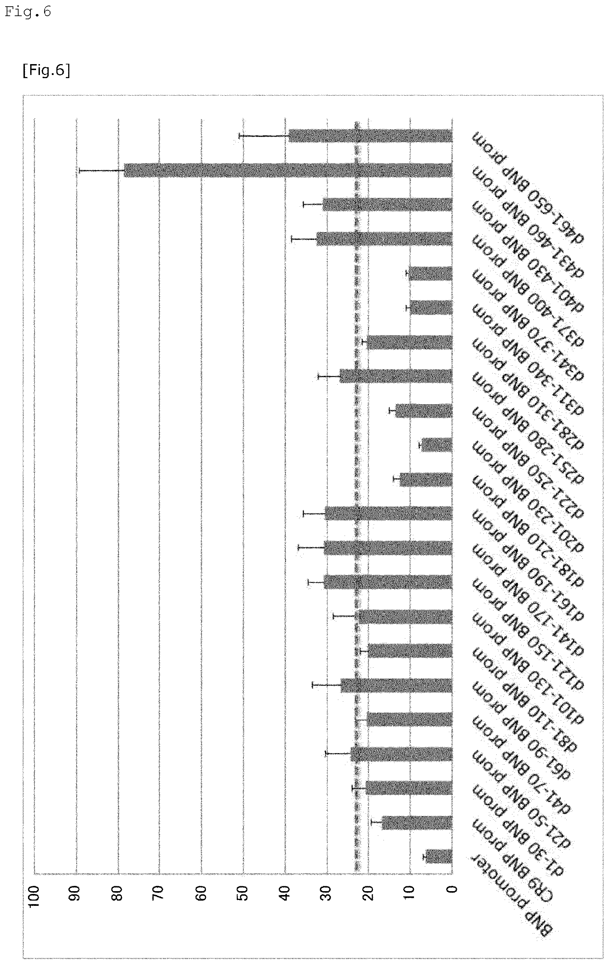

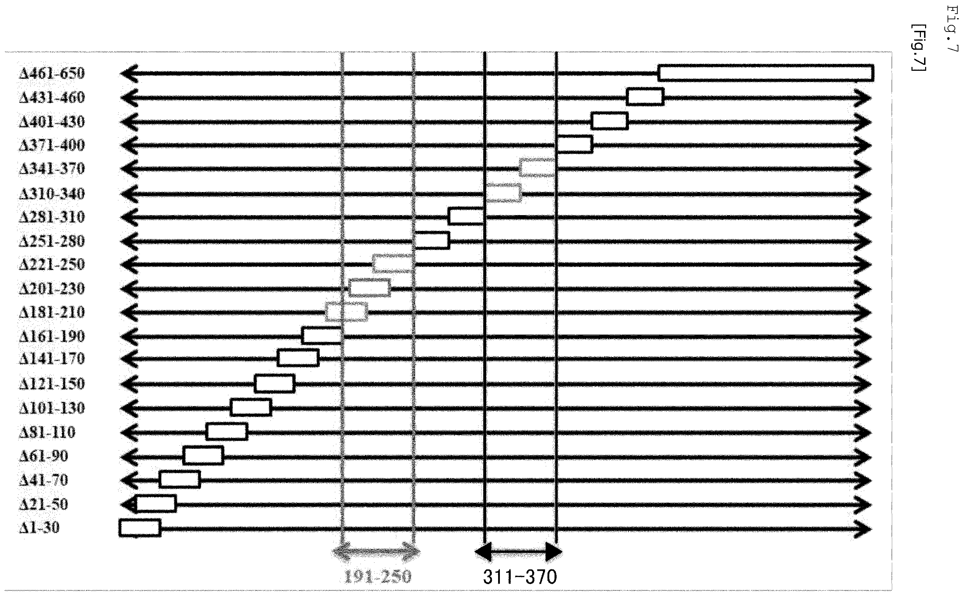

Results of the luciferase assay are shown in Fig. 6. In addition, Fig. 7 shows a summary of the results of the luciferase assay. Deletion Lentivector in which the 191-250 or 311-370 region is deleted had reduced reactivity to Phenylephrine.

Sequence of the 191-250th region (SEQ ID NO: 8: hereinafter, also referred to as "191-250 region") and sequence of the 311-370th region (SEQ ID NO: 7: hereinafter, also referred to as "311-370 region") in SEQ ID NO: 12 are represented below. The 191-250 region and 311-370 region are shown in uppercase.

ctcatggtgccagttgctgcccttggccaaaaacatgtcctcttcatccagcttcataaacacggcagggacagaacttgccacaccgaaac
ccatacatggtctggagactgcacaccaactgggatcacagggacgcgcaggggggttcacctcctccaaggaagaggcagggatagga
gtgaaaccagGGAGCTATTTCGAGAAGGTGTCAAAGGCCGGAACACTAAAATTAGAGCCT
TGGTTCCTAAaaagacattagcaccaaccgaggtctaagagaggggctcttcagcgcggaatgtgaccccAGGAAGCG
CTCCGGCGATCCTGGGCAATGAGGTCAGCAGGACCAATGGGACCCTCCACCTcccttatg
gtcacgtggtggtcatggggacctctccccgagccagctctggggctgtttccaaccacttcccgtgtacaccaagccaggaaaggcaag
caggcagagctgctgtccgtgtagggctgaccttctgggctgtgccttttctttttgattgtcctctgtgctttctgttccattctcagtttaagagt
gggagaggaggatgcttggaagaggcagccatcttggaacctggtggtttggcctgggagataaaccaggaagggcctaggat (SEQ
ID NO: 12)

### 2. Verification of functions of the 191-250 region and 311-370 region

### 2-1. Preparation of vector comprising enhancer polynucleotide of Example 1

In order to verify that the 191-250 region and the 311-370 region in the CR9 region are important for expression of ANP and BNP, an enhancer polynucleotide in which the 191-250 region and the 311-370 region are artificially connected to each other (Example 1) was prepared and its enhancer activity was measured.

The CR9 650 bp comprised in the original vector prepared in the above II. 1-1. was replaced with the enhancer polynucleotide (Example 1) having the sequence represented below of about 120 bp to prepare a 191-250/311-370 reporter vector. The lowercase part indicates a restriction enzyme site connecting the 191-250 region and the 311-370 region.

GGAGCTATTTCGAGAAGGTGTCAAAGGCCGGAACACTAAAATTAGAGCCTTGGTTC
CTAAactagAGGAAGCGCTCCGGCGATCCTGGGCAATGAGGTCAGCAGGACCAATGGG
ACCCTCCACCT (SEQ ID NO: 11)

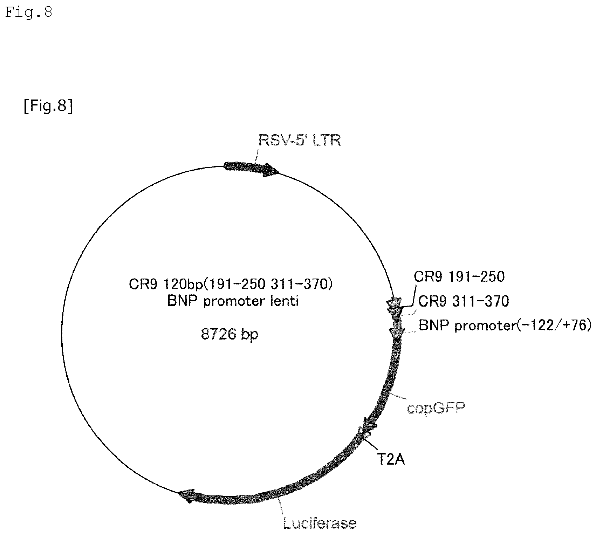

Fig. 8 shows a map of the 191-250/311-370 reporter vector.

In order to perform a comparative experiment, a BNP promoter vector obtained by removing the sequence of the CR9 region from the original vector, a 191-250 reporter vector obtained by replacing the CR9 650 bp comprised in the original vector with only the 191-250 region, and a 311-370 reporter vector obtained by replacing the CR9 650 bp comprised in the original vector with only the 191-250 region were prepared.

### 2-2. Luciferase assay

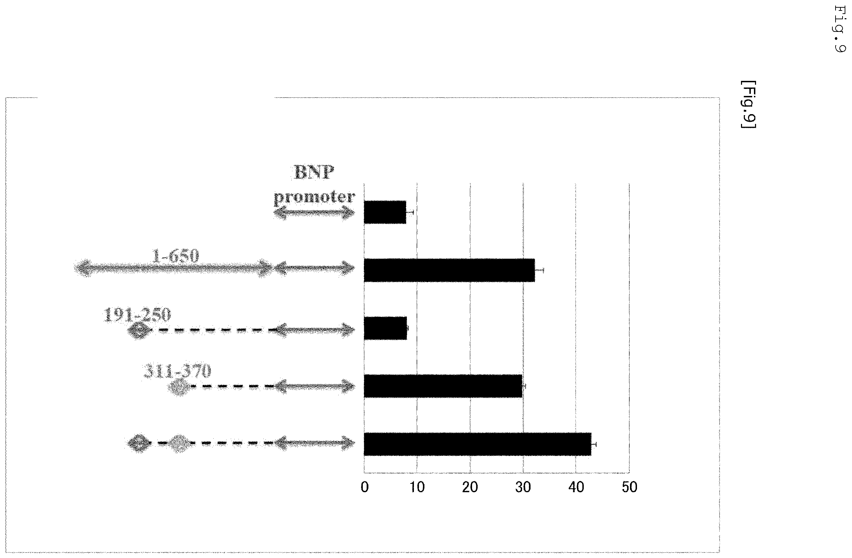

Luciferase assay was performed in the same manner as the above II. 1-2. Its results are shown in Fig. 9. Luciferase activity of the original vector was higher than that of the BNP promoter vector. Luciferase activity of the 191-250/311-370 reporter vector was higher than that of the original vector. The 311-370 reporter vector had luciferase activity equivalent to that of the original vector. On the other hand, the 191-250 reporter vector was comparable to the BNP promoter vector, and its enhancer activity was weak.

The above results show that, for enhancing the activity of the BNP promoter, the enhancer activity of the enhancer having both of the 191-250 region and the 311-370 region is stronger. In addition, the length of nucleotide of the enhancer having the 191-250 region and the 311-370 region is shorter than that of the CR9 650 bp, but its enhancer activity is higher than that of the CR9 650 bp.

### III. Experimental example 3: Analysis of the region responding to heart failure in the CR9 region of a human ANP/BNP gene

### 1. Sequence of the CR9 region of the human ANP/BNP gene

The human CR9 region is a 676 bp region represented by the following sequence.

gtgctgtgagctgccctcccagaggtgagttctctgggtcctgccacacaaaacacatggcaggggcgtggatccctacacccaaacccat
acaaggccttggacctgtgctcaccaactcagatcacggggatgcaggggggttcaccttctccaaggaagggccagggacacaagagaa
aacagGGAGCTATTTTGAGAAGGTGTCAAGGCTGGGACACTAAAATTAGAGGCTTGGT
TCCTAAaaagacattggcgccaaccgaggccttgaagaggggctcttcagcacggaatgtgaccccAGGAAGCACGCT
CACAATTCTGGACAATGAGGTCAGCCTGCAGGGCGCATGGACCGCCCAatggccggtgg
gcatgggcacctctcccccgccagccccacctgcagaatgctggaactggctccaaccgcttcccgtctgcactgagccagcgcaagg
gcgagcggacagagcctcatgttcccggtgctgctgcccttccaggctgccttcctttttgactgtccttttggcttcttatgcttttggctccatt
ctggatgtgcgtcatggggtgggatgcagaagcagcagccagcttggaggctggtgttcccggaggctgagggcgggatgggggaaa
tatgaaccaggaaagggcctggtat (SEQ ID NO: 13)

In addition, Fig. 10 shows alignment between the mouse CR9 region and the human CR9 region.

In the human CR9 region, a region having high identity with the mouse 191-250 region was a sequence of the 186-244th region (SEQ ID NO: 5: hereinafter also referred to as "186-244 region"). There were 5 base mismatches between the 191-250 region and the 186-244 region, with an identity of approximately 91.5%. In addition, in the human CR9 region, the portion having high identity with the mouse 311-370 region was a sequence of the 305-365th region (SEQ ID NO: 4: hereinafter, also referred to as "305-365 region"). There were 16 base mismatches between the 311-370 region and the 305-365 region, with an identity of approximately 73.8%. In the above sequence, the 186-244 region and the 305-365 region are shown in uppercase, and the other regions are shown in lowercase.

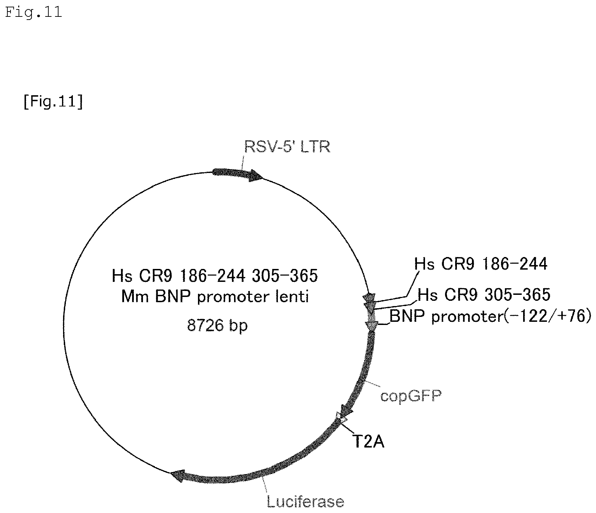

Fig. 11 shows a map of a 186-244/305-365 reporter vector.

The CR9 650 bp comprised in the original vector prepared in the above II. 1-1. was replaced with an enhancer polynucleotide (Example 2) having a sequence represented below of about 120 bp to prepare the 186-244/305-365 reporter vector. The lowercase part indicates a restriction enzyme site connecting the 186-244 region with the 305-365 region.

GGAGCTATTTGAGAAGGTGTCAAGGCTGGGACACTAAAATTAGAGGCTTGGTTCCT
AAgaattcAGGAAGCACGCTCACAATTCTGGACAATGAGGTCAGCCTGCAGGGCGCAT
GGGACCGCCCA (SEQ ID NO: 10)

In addition, in order to perform a comparative experiment, a Hs 1-676 vector obtained by replacing the CR9 650 bp comprised in the original vector of mouse with the human CR9-650 represented by SEQ ID NO: 13, a 186-244 reporter vector obtained by replacing the CR9 650 bp comprised in the original vector with only the human 186-244 region, and a 305-365 reporter vector obtained by replacing the CR9 650 bp comprised in the original vector with only the human 305-365 region were prepared.

### 2. Luciferase assay

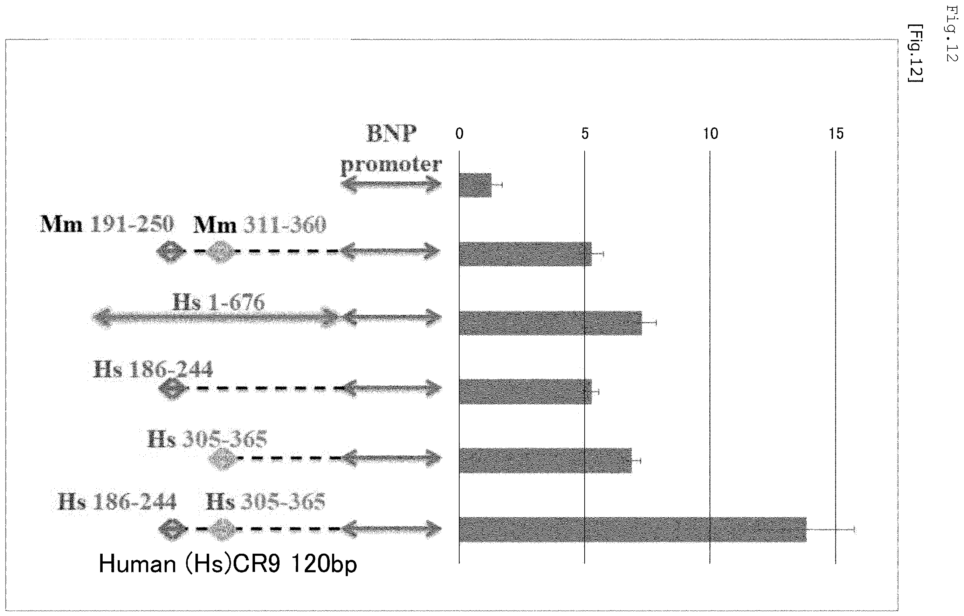

Luciferase assay was performed in the same manner as the above II. 1-2. Its results are shown in Fig. 12. A BNP promoter vector was used as a negative control for enhancer activity. In addition, the 191-250/311-370 reporter vector of mouse was used as a positive control for enhancer activity. The Hs 1-676 vector had luciferase activity higher than that of the 191-250/311-370 reporter vector. The 186-244 reporter vector had luciferase activity equivalent to that of the 191-250/311-370 reporter vector. The 305-365 reporter vector had luciferase activity higher than that of the 186-244 reporter vector. The 186-244/305-365 reporter vector had the highest luciferase activity.

It was found from the above results that, the CR9 region of human had higher enhancer activity as compared with that of mice. In particular, it was found that the enhancer polynucleotide of the Example 2 had further higher enhancer activity.

### IV. Experimental example 4: Specification of enhancer sequence by deletion mutants

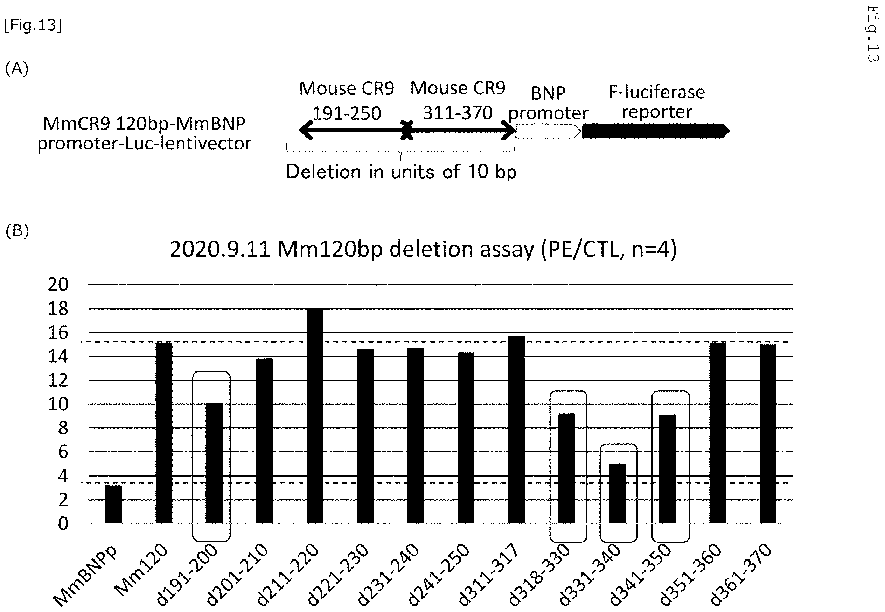

Deletion mutants were prepared by deleting a part of the 191-250 and the 311-370 regions of the reporter vector of Example 1 shown in Experimental Example 2 in units of 10 bp. A design of its construct is shown in Fig. 13A. Luciferase assay was performed for each of the deletion mutants, and activity as an enhancer was evaluated. The luciferase assay was performed in the same manner as the above II. 1-2.

The results are shown in Fig. 13B. When 191-200 region, 318-330 region, 331-340 region, or 341-350 region was deleted, decreased transcriptional activity was shown. In particular, the transcriptional activity was remarkably decreased in the deletion of the 331-340 region.

Next, deletion mutants obtained by deleting a part of the 318-330 region, the 331-340 region, and the 341-350 region in units of 4 bp were prepared, and luciferase assay was performed to evaluate activity as an enhancer.

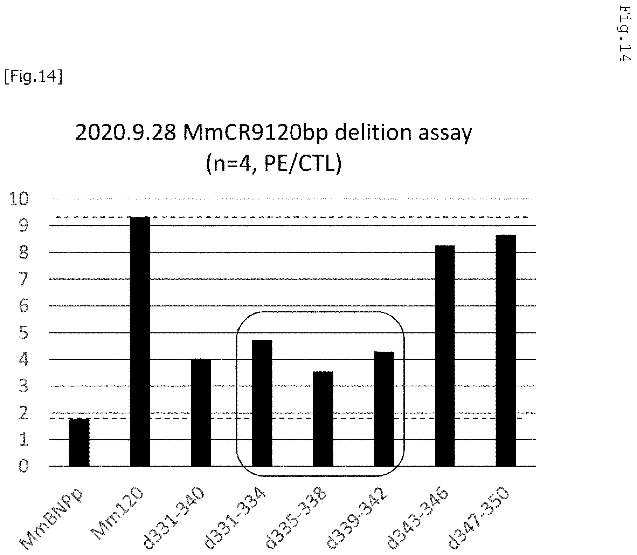

Fig. 14 shows the results in the deletion mutants obtained by deleting a part of the 318-350 region in units of 4 bp. In the case of deletion of the 331-334 region, the 335-338 region, or the 339-342 region, decreased transcriptional activity was shown.

Fig. 15 shows 331-342 region, with a broken line, in which enhancer activity was conserved as shown in the experiment where a part of the 318-350 region was deleted in units of 4 bp. It was a region where only 1 bp difference was observed between mouse and human, and it was very well conserved.

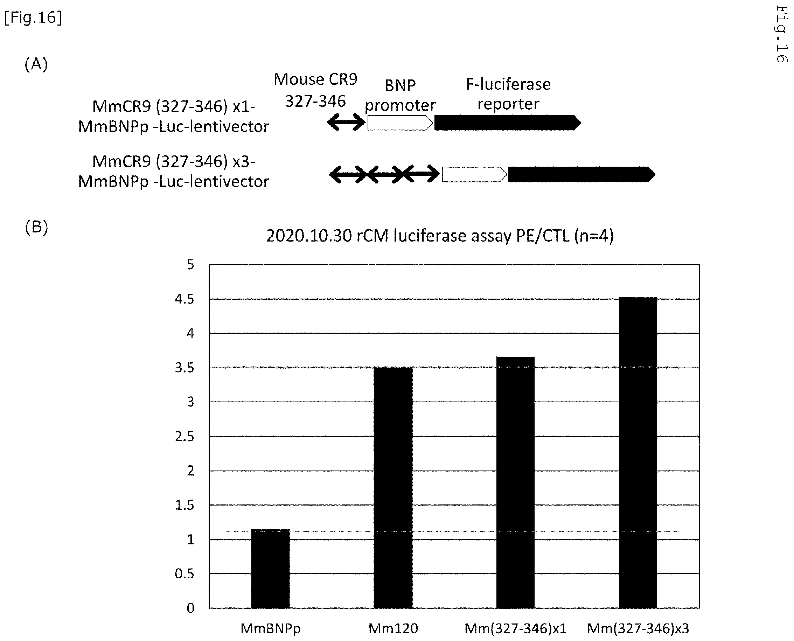

Next, a construct in which 327-346 region including the sequence of the mouse 331-342 region was inserted into the reporter vector instead of the sequence of Example 1, and a construct in which a sequence obtained by repeating the sequence of the 327-346 region including the sequence of the mouse 331-342 region three times was inserted into the reporter vector instead of the sequence of Example 1 were prepared. Fig. 16A shows designs of each construct. Luciferase assay was performed for each construct, and activity as an enhancer was evaluated. The luciferase assay was performed in the same manner as the above II. 1-2.

The results are shown in Fig. 16 B. MmBNPp indicates a reporter vector that does not have an enhancer sequence. Mm120 shows the construct of Example 1. Mm (327-346) × 1 indicates the construct into which only one sequence of the 327-346 region is inserted. Mm (327-346) × 3 indicates the construct into which the sequence of the 327-346 region is inserted in tandem three times. Mm (327-346) × 1 had slightly higher enhancer activity than Example 1. In addition, Mm (327-346) × 3 had higher activity than Mm (327-346) × 1. Therefore, it can be considered that this region is the core of enhancer activity. This region corresponds to the 325-336 region of the human CR9 region.

### V. Experimental example 5: Transcriptional activity of 186-244/305-365 reporter vector in hiPS-derived cardiomyocytes

Enhancer activity of a BNP promoter vector comprising 186-244 region and 305-365 region (also referred to as HsCR9 120bp) in human cardiomyocytes was verified.

### (1) Method

The reporter vector infected cardiomyocytes differentiated from human iPS cells instead of the cells used in Experimental Example 3, and the expression levels of mRNA of Nppb, luciferase, and GAPDH were measured by real-time RT-PCR.

Specifically, hiPS-derived cardiomyocytes after differentiation ware plated on a 12-well plate so as to be 1×10⁵ cells/well, and 24 hours later, were infected with two types: (i) BNP promoter only and (ii) HsCR9 120bp + BNP promoter of luciferase reporter lentivector. From 48 hours after plating, phenylephrine loading (100 µM) was performed for 24 hours. 72 hours after plating, cells were harvested and RNA was extracted. Luciferase gene expression and endogenous Nppb gene expression were evaluated by quantitative PCR.

### (2) Results

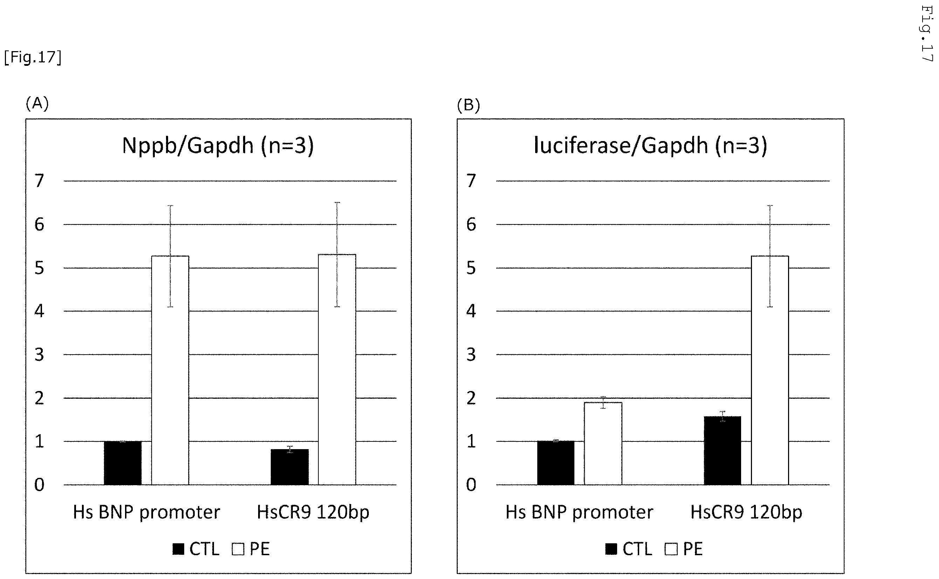

The results are shown in Fig. 17. Fig. 17A shows a relative expression level of Nppb to an expression level of GAPDH. Fig. 17B shows a relative expression level of luciferase to the expression level of GAPDH. In hiPS-derived cardiomyocytes infected with any of the lentivectors, an increase in Nppb gene expression was observed by loading phenylephrine. In the lentivector into which the human CR9 120 bp was inserted, luciferase expression was more strongly induced by loading phenylephrine.

These results indicate that the human CR9 120 bp enhancer sequence is responsive to heart failure in the myocardium.

### VI. Experimental example: 6 : Verification of heart-specific enhancer activity in vivo

Four types (120 bp enhancer (+) or (-), cTnT promoter or BNP promoter) of AAV6-BNP-T2A-luciferase reporter vectors were administered to the CR9 KO mice prepared in Experimental Example 1, and cardiac specificity and heart failure responsiveness of luciferase expression were evaluated by live imaging.

### (1) Method

### 1) Vector

Two types of AAV6-BNP-T2A-luciferase reporter vectors, i.e., AAV6-BNP-T2A-Luciferase reporter vector into which the mouse enhancer polynucleotide shown in Example 1 is inserted and AAV6-BNP-T2A-luciferase reporter vector into which the mouse enhancer polynucleotide shown in Example 1 (hereinafter referred to as "MmCR9 120bp") is not inserted, were prepared. Furthermore, a total of four types of AAV6-BNP-T2A-luciferase reporter vectors shown below were prepared by inserting the cTnT promoter or the BNP promoter as a promoter sequence into each of these AAV6-BNP-T2A-luciferase reporter vectors.
i. AAV6_(cTnT promoter)_nFLAG MmBNP_T2A_FLuc
ii. AAV6_MmCR9 120bp (cTnT promoter)_nFLAG MmBNP_T2A_FLuc
iii. AAV6_(BNP promoter)_nFLAG MmBNP_T2A_FLuc
iv. AAV6_MmCR9 120bp (BNP promoter)_nFLAG MmBNP_T2A_FLuc

### 2) Gene transfer

Each AAV6-BNP-T2A-luciferase reporter vector was intravenously administered to CR9 KO mice from orbital sinus so as to be 1 × 10¹¹ vg.

### 3) Evaluation

After administration of each AAV6-BNP-T2A-luciferase reporter vector, luciferase expression was evaluated by live imaging with the elapse of time with IVIS Lumina II ^{™} on day 13, day 27, and day 34.

IVIS Lumina II ^{™} (Caliper Life Sciences) was used as an imaging device, and Living Image 4.0 (Caliper Life Sciences) was used as an analysis software. At the time of imaging, mice were anesthetized by inhalation with ISOFLURANE Inhalation Solution ^{™}, and their hair was removed from their neck, chest, and abdomen. D-Luciferin (VivoGlo Luciferin In Vivo Grade ^{™}, Promega), which is a luminescent substrate, was intravenously administered to the mice so as to be 75 mg/kg, and the mice were fixed on their backs in an imaging device under anesthesia. Images were taken for 20 seconds from 1 minute after administration of the D-Luciferin.

In addition, continuous subcutaneous administration of Phenylephrine (75 mg/kg/day, osmotic minipump (Alzet, Cupertino, CA)) was started on day 34 to cause drug-loaded heart failure, and luciferase expression was evaluated by live imaging on day 48 and day 60.

After euthanizing the mice, the organs were excised and ex-vivo imaging was performed. Furthermore, DNA and RNA were extracted from the excised organs, and the amount of AAV6 virus infected and the expression of luciferase were evaluated by quantitative PCR.

### (2) Results

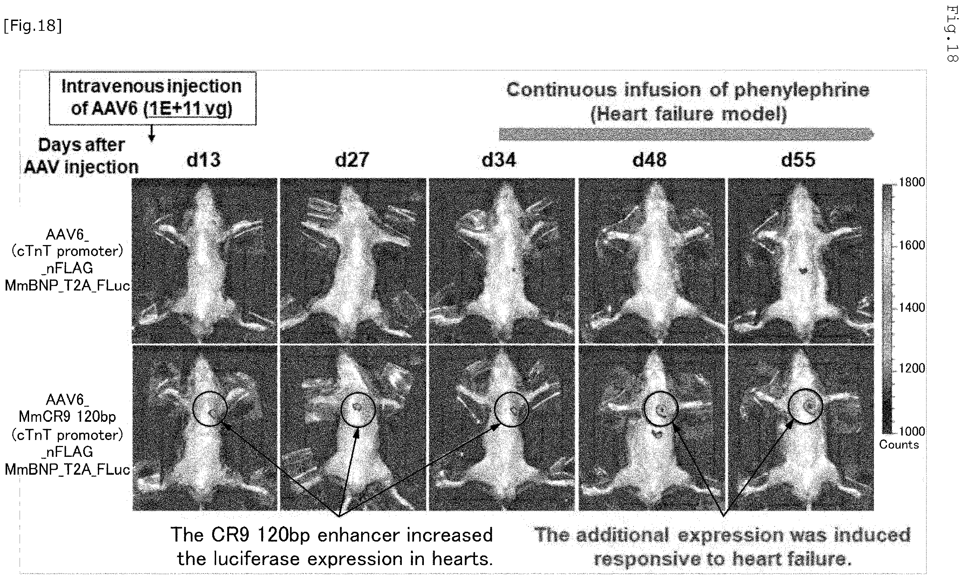

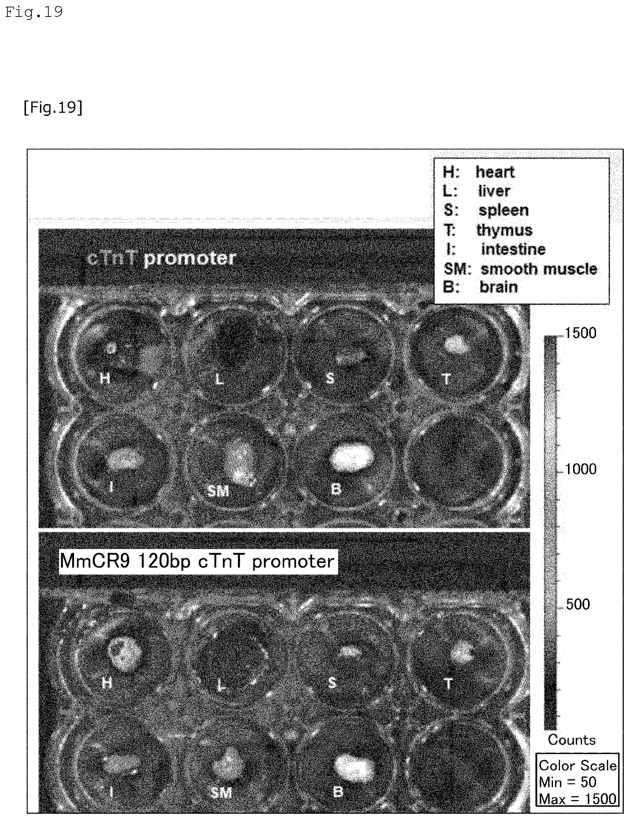

Fig. 18 shows captured images of luminescence in i: AAV6_(cTnT promoter)_nFLAG MmBNP_T2A_FLuc-transferred mouse (upper) and ii: AAV6_MmCR9 120bp (cTnT promoter)_nFLAG MmBNP_T2A_FLuc-transferred mouse (lower). As shown in Fig. 18, strong luminescence was detected in the AAV6_MmCR9 120bp (cTnT promoter)_nFLAG MmBNP_T2A_FLuc-transferred mouse near its heart, but luminescence was not detected in the AAV6_(cTnT promoter)_nFLAG MmBNP_T2A_FLuc-transferred mouse not comprising MmCR9 120bp. In addition, Fig. 19 shows captured images of luminescence in hearts, livers, spleens, thymuses, intestines, smooth muscles, and brains excised from each mouse. Stronger luminescence was detected in the AAV6_MmCR9 120bp (cTnT promoter)_nFLAG MmBNP_T2A_FLuc-transferred mouse compared with the AAV6_(cTnT promoter)_nFLAG MmBNP_T2A_FLuc-transferred mouse. Especially after causing drug-loaded heart failure, the enhancement of luminescence by MmCR9 120bp was remarkable.

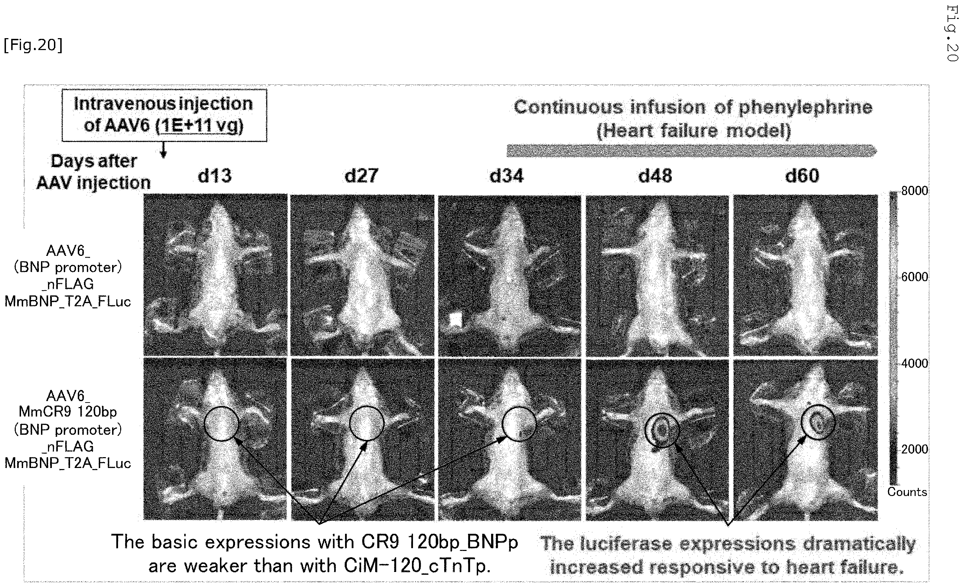

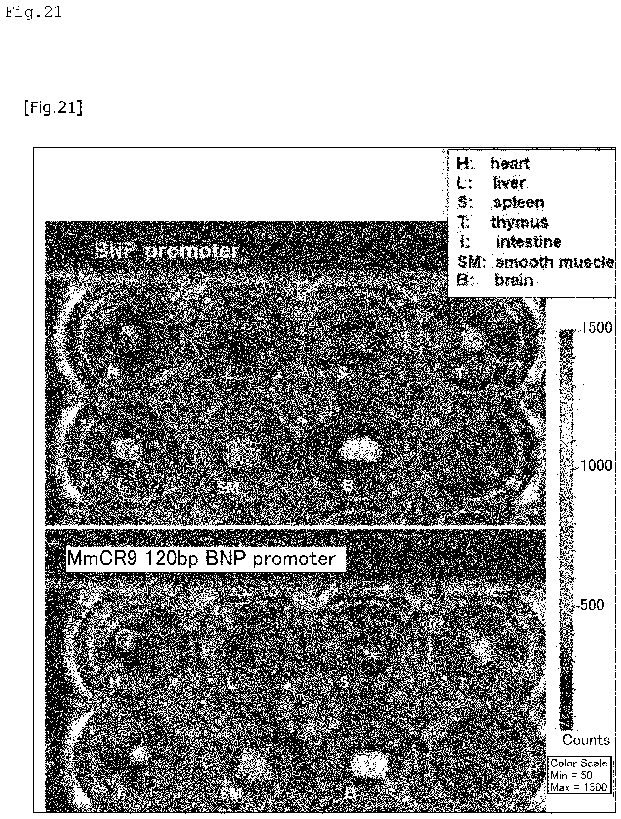

Fig. 20 shows captured images of luminescence in iii: AAV6_(BNP promoter)_nFLAG MmBNP_T2A_Fluc-transferred mouse (upper) and iv: AAV6_MmCR9 120bp (BNP promoter)_nFLAG MmBNP_T2A_FLuc-transferred mouse (lower). As shown in Fig. 20, strong luminescence was detected in the AAV6_MmCR9 120bp (BNP promoter)_nFLAG MmBNP_T2A_FLuc-transferred mouse near its heart after causing drug-loaded heart failure, but luminescence was not detected in the AAV6_(BNP promoter)_nFLAG MmBNP_T2A_Fluctransferred mouse not comprising MmCR9 120bp. In addition, Fig. 21 shows captured images of luminescence in hearts, livers, spleens, thymuses, intestines, smooth muscles, and brains excised from each mouse. Stronger luminescence was detected in the AAV6_MmCR9 120bp (BNP promoter)_nFLAG MmBNP_T2A_FLuc-transferred mouse compared with the AAV6_(BNP promoter)_nFLAG MmBNP_T2A_FLuc-transferred mouse. Especially after causing drug-loaded heart failure, the enhancement of luminescence by MmCR9 120bp was remarkable.

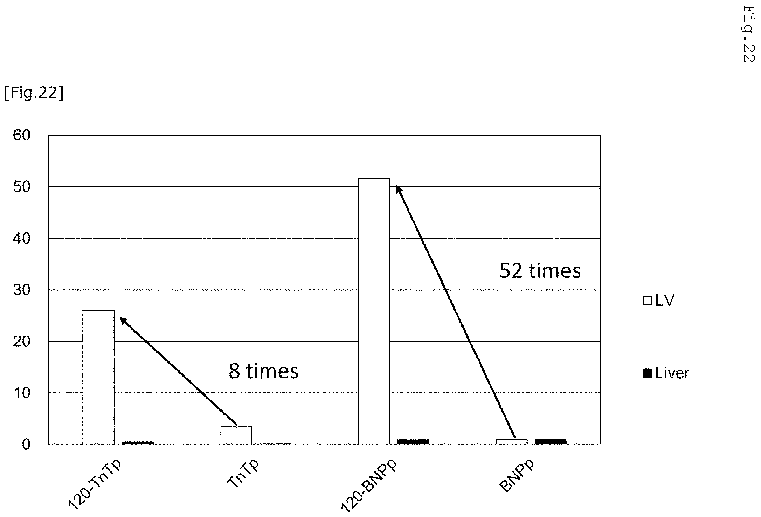

Here, as shown in Figs. 19 and 21, luminescence was observed not only in the hearts but also in the livers. In order to verify whether this indicates the effect of the enhancers in a liver, the amount of AAV6 DNA and the expression levels of luciferase in a liver and a heart were measured. The results are shown in Fig. 22. In Fig. 22, TnTp shows organs excised from the AAV6_(cTnT promoter)_nFLAG MmBNP_T2A_FLuc-transferred mouse, 120-TnTp shows organs excised from the AAV6_MmCR9 120bp (cTnT promoter)_nFLAG MmBNP_T2A_FLuctransferred mouse, BNPp shows organs excised from the AAV6_(BNP promoter)_nFLAG MmBNP_T2A_FLuc-transferred mouse, and 120-BNPp shows organs excised from the AAV6_MmCR9 120bp (BNP promoter)_nFLAG MmBNP_T2A_FLuc-transferred mouse. In addition, the graph shows relative expression levels of luciferase mRNA to the amount of AAV6 DNA.

In the hearts (left ventricle: LV) of 120-TnTp and 120-BNPp, the relative expression level of luciferase mRNA was increased depending on the presence or absence of MmCR9 120 bp (8-fold and 52-fold, respectively), but in the livers, the expression of luciferase mRNA was not detected. From this, it was considered that the luminescence in the heart is due to the expression of luciferase that depends on the enhancer, but the luminescence in the liver is a non-specific luminescence that does not depend on the enhancer.

From the above results, it was shown that the enhancer polynucleotide according to the present invention exhibits enhancer activity in a heart-specific manner, and the enhancer activity is increased in response to heart failure.

## Claims

1. An enhancer polynucleotide comprising
a polynucleotide consisting of a sequence having 80% or more identity with a sequence represented by SEQ ID NO: 1, or
a polynucleotide consisting of a sequence having 80% or more identity with a sequence represented by SEQ ID NO: 2
wherein the enhancer polynucleotide responds to heart failure (where the enhancer polynucleotide excludes a polynucleotide having the same sequence as a sequence represented by SEQ ID NO: 13 and a polynucleotide having the same sequence as a sequence represented by SEQ ID NO: 12).

2. The enhancer polynucleotide according to Claim 1, wherein
the enhancer polynucleotide comprising the polynucleotide consisting of the sequence having 80% or more identity with the sequence represented by SEQ ID NO: 1 consists of a sequence having 70% or more identity with a sequence represented by SEQ ID NO: 4, or
the enhancer polynucleotide comprising a polynucleotide consisting of the sequence having 80% or more identity with the sequence represented by SEQ ID NO: 2 consists of a sequence having 70% or more identity with a sequence represented by SEQ ID NO: 7.

3. The enhancer polynucleotide according to Claim 1 or 2, wherein
(i) the sequence having 80% or more identity with the sequence represented by SEQ ID NO: 1 or
(ii) the sequence having 80% or more identity with the sequence represented by SEQ ID NO: 2
is a sequence represented by SEQ ID NO: 3.

4. The enhancer polynucleotide according to any one of Claims 1 to 3, wherein the enhancer polynucleotide further comprises
a polynucleotide consisting of a sequence having 90% or more identity with a sequence represented by SEQ ID NO: 5 or
a polynucleotide consisting of a sequence having 90% or more identity with a sequence represented by SEQ ID NO: 8.

5. The enhancer polynucleotide according to any one of Claims 1 to 4, wherein the enhancer polynucleotide is
A: obtained by connecting (i) the polynucleotide consisting of the sequence having 80% or more identity with the sequence represented by SEQ ID NO: 1, and (ii) the polynucleotide consisting of a sequence having 90% or more of the sequence represented by SEQ ID NO: 5 directly or via an adapter, or
B: obtained by connecting (iii) the polynucleotide consisting of the sequence having 80% or more identity with the sequence represented by SEQ ID NO: 2 and (iv) the polynucleotide consisting of the sequence having 90% or more identity with the sequence represented by SEQ ID NO: 8 directly or via an adapter, and wherein
the length of the adapter is 10 nucleotides or less when expressed in terms of the number of nucleotides in a single-stranded polynucleotide.

6. The enhancer polynucleotide according to Claim 1, wherein the total length of the enhancer polynucleotide is 200 nucleotides or less when expressed in terms of the number of nucleotides in the single-stranded polynucleotide.

7. An enhancer polynucleotide comprising a polynucleotide consisting of the sequence represented by SEQ ID NO: 10 or SEQ ID NO: 11, wherein the enhancer polynucleotide responds to heart failure.

8. An enhancer polynucleotide comprising a reverse complementary sequence of the enhancer polynucleotide according to any one of Claims 1 to 7, wherein the enhancer polynucleotide responds to heart failure.

9. An expression vector comprising a polynucleotide containing a promoter sequence and the enhancer polynucleotide according to any one of Claims 1 to 8, wherein the polynucleotide containing a promoter sequence and the enhancer polynucleotide are connected so that the enhancer polynucleotide enhances the transcriptional activity of the promoter polynucleotide.

10. A composition for gene therapy comprising a polynucleotide in which the polynucleotide to be expressed is integrated into the expression vector according to Claim 9 so as to be expressible.

11. The composition according to Claim 10 for preventing and/or treating heart failure.

12. A composition for preventing and/or treating heart failure, comprising
a polynucleotide comprising an sgRNA sequence that binds to a polynucleotide consisting of the sequence represented by SEQ ID NO: 1, the sequence represented by SEQ ID NO: 2, the sequence represented by SEQ ID NO: 4, the sequence represented by SEQ ID NO: 5, the sequence represented by SEQ ID NO: 7 or the sequence represented by SEQ ID NO: 8, or a polynucleotide consisting of a complementary sequence thereof, or
a vector capable of expressing a crRNA that binds to the polynucleotide consisting of the sequence represented by SEQ ID NO: 1, the sequence represented by SEQ ID NO: 2, the sequence represented by SEQ ID NO: 4, the sequence represented by, SEQ ID NO: 5, the sequence represented by SEQ ID NO: 7 or the sequence represented by SEQ ID NO: 8, or the polynucleotide consisting of the complementary sequence thereof.

13. A kit for preventing and/or treating heart failure comprising the composition according to Claim 12.


### Sequence Listing

SEQUENCE LISTING

<110>  OSAKA UNIVERSITY

<120>  An enhancer polynucleotide responding to heart failure, and an
       expression vector containing the enhancer polynucleotide

<130>  P20-213WO

<150>  JP 2019-206823
<151>  2019-11-15

<160>  16

<170>  PatentIn version 3.5

<210>  1
<211>  12
<212>  DNA
<213>  Homo sapiens

<400>  1
tggacaatga gg                                                          12


<210>  2
<211>  12
<212>  DNA
<213>  Mus musculus

<400>  2
tgggcaatga gg                                                          12


<210>  3
<211>  12
<212>  DNA
<213>  Artificial Sequence

<220>
<223>  enhancer


<220>
<221>  n
<222>  (4)..(4)
<223>  n is a, g, t, or c

<220>
<221>  misc_feature
<222>  (4)..(4)
<223>  n is a, c, g, or t

<400>  3
tggncaatga gg                                                          12


<210>  4
<211>  61
<212>  DNA
<213>  Homo sapiens

<400>  4

aggaagcacg ctcacaattc tggacaatga ggtcagcctg cagggcgcat gggaccgccc 60

a 61

<210> 5
<211> 59
<212> DNA
<213> Homo sapiens

<400> 5
ggagctattt tgagaaggtg tcaaggctgg gacactaaaa ttagaggctt ggttcctaa 59

<210> 6
<211> 60
<212> DNA
<213> Homo sapiens

<400> 6
aaagacattg gcgccaaccg aggccttgaa gaggggctct tcagcacgga atgtgacccc 60

<210> 7
<211> 60
<212> DNA
<213> Mus musculus

<400> 7
aggaagcgct ccggcgatcc tgggcaatga ggtcagcagg accaatggga ccctccacct 60

<210> 8
<211> 60
<212> DNA
<213> Mus musculus

<400> 8
ggagctattt cgagaaggtg tcaaaggccg gaacactaaa attagagcct tggttcctaa 60

<210> 9
<211> 60
<212> DNA
<213> Mus musculus

<400> 9
aaagacatta gcaccaaccg aggtctaaga gaggggctct tcagcgcgga atgtgacccc 60

<210> 10
<211> 126
<212> DNA
<213> Artificial Sequence

<220>
<223> enhancer

<400> 10
ggagctattt tgagaaggtg tcaaggctgg gacactaaaa ttagaggctt ggttcctaag 60

aattcaggaa gcacgctcac aattctggac aatgaggtca gcctgcaggg cgcatgggac 120

cgccca                                                                 126


<210>  11
<211>  125
<212>  DNA
<213>  Artificial Sequence

<220>
<223>  enhancer

<400>  11
ggagctattt cgagaaggtg tcaaaggccg gaacactaaa attagagcct tggttcctaa      60

actagaggaa gcgctccggc gatcctgggc aatgaggtca gcaggaccaa tgggaccctc     120

cacct                                                                 125


<210>  12
<211>  650
<212>  DNA
<213>  Mus musculus

<400>  12
ctcatggtgc cagttgctgc ccttggccaa aaacatgtcc tcttcatcca gcttcataaa      60

cacggcaggg acagaacttg ccacaccgaa acccatacat ggtctggaga ctgcacacca     120

actgggatca cagggacgcg cagggggttc acctcctcca aggaagaggc agggatagga     180

gtgaaaccag ggagctattt cgagaaggtg tcaaaggccg gaacactaaa attagagcct     240

tggttcctaa aaagacatta gcaccaaccg aggtctaaga gaggggctct tcagcgcgga     300

atgtgacccc aggaagcgct ccggcgatcc tgggcaatga ggtcagcagg accaatggga     360

ccctccacct cccttatggt cacgtggtgg tcatggggac ctctccccga gccagctctg     420

gggctgtttc caaccacttc ccgtgtacac caagccagga aaggcaagca ggcagagctg     480

ctgtccgtgt agggctgacc ttctgggctg tgccttttct tttttgattg tcctctgtgc     540

tttctgttcc attctcagtt taagagtggg agaggaggat gcttggaaga ggcagccatc     600

ttggaacctg gtggtttggc ctgggagata aaccaggaag ggcctaggat                650


<210>  13
<211>  676
<212>  DNA
<213>  Homo sapiens

<400>  13
gtgctgtgag ctgccctccc agaggtgagt tctctgggtc ctgccacaca aaacacatgg      60

caggggcgtg gatccctaca cccaaaccca tacaaggcct tggacctgtg ctcaccaact     120

cagatcacgg ggatgcaggg ggttcacctt ctccaaggaa gggccaggga cacaagagaa     180

aacagggagc tattttgaga aggtgtcaag gctgggacac taaaattaga ggcttggttc     240

ctaaaaagac attggcgcca accgaggcct tgaagagggg ctcttcagca cggaatgtga     300

ccccaggaag cacgctcaca attctggaca atgaggtcag cctgcagggc gcatgggacc 360

gcccaatggc cggtgggcat gggcacctct ccccccgcca gccccacctg cagaatgctg 420

gaactggctc caaccgcttc ccgtctgcac tgagccagcg caagggcgag cggacagagc 480

ctcatgttcc cggtgctgct gcccttccag gctgccttcc ttttttgact gtccttttgg 540

cttcttatgc ttttggctcc attctggatg tgcgtcatgg ggtgggatgc agaagcagca 600

gccagcttgg aggctggtgt tcccggaggc tgagggcggg atggggggaa atatgaacca 660

ggaaagggcc tggtat 676

<210> 14
<211> 198
<212> DNA
<213> Mus musculus

<400> 14
cctgagctca gccggcagga atgcagctga taaatcagag ataaccccac ccctactccg 60

tgaaaaggtc tggccggaca ctcagcccca gtataaaagg cagaggcacc gttgttgaag 120

acaccagtgc acaagctgct tggggaggcg agacaaggga gaacacggca tcattgcctg 180

gcccatcgct tctgcggc 198

<210> 15
<211> 460
<212> DNA
<213> Mus musculus

<400> 15
acaccgaaac ccatacatgg tctggagact gcacaccaac tgggatcaca gggacgcgca 60

gggggttcac ctcctccaag gaagaggcag ggataggagt gaaaccaggg agctatttcg 120

agaaggtgtc aaaggccgga acactaaaat tagagccttg gttcctaaaa agacattagc 180

accaaccgag gtctaagaga ggggctcttc agcgcggaat gtgaccccag gaagcgctcc 240

ggcgatcctg ggcaatgagg tcagcaggac caatggaccc ctccacctcc cttatggtca 300

cgtggtggtc atggggacct ctccccgagc cagctctggg gctgtttcca accacttccc 360

gtgtacacca agccaggaaa ggcaagcagg cagagctgct gtccgtgtag ggctgacctt 420

ctgggctgtg cctttctttt tttgattgtc ctctgtgctt 460

<210> 16
<211> 466
<212> DNA
<213> Homo sapiens

<400> 16
acacccaaac ccatacaagg ccttggacct gtgctcacca actcagatca cggggatgca 60

gggggttcac cttctccaag gaagggccag ggacacaaga gaaaacaggg agctattttg 120

agaaggtgtc aaggctggga cactaaaatt agaggcttgg ttcctaaaaa gacattggcg 180

ccaaccgagg ccttgaagag gggctcttca gcacggaatg tgaccccagg aagcacgctc 240

acaattctgg acaatgaggt cagcctgcag ggcgcatggg accgcccaat ggccggtggg 300

catgggcacc tctccccccg ccagccccac ctgcagaatg ctggaactgg ctccaaccgc 360

ttcccgtctg cactgagcca gcgcaagggc gagcggacag agcctcatgt tcccggtgct 420

gctgcccttc caggctgcct tccttttttg actgtccttt tggctt 466